# EUROPEAN PATENT APPLICATION

(11) **EP 4 593 036 A1**
(43) Date of publication of application: **30.07.2025**
(21) Application number: 24837830.9
(22) Date of filing: 19.07.2024
(51) Int. Cl.: G16H 50/20

(54) **DATA PROCESSING METHOD, ELECTRONIC DEVICE, WEARABLE DEVICE AND COMMUNICATION SYSTEM**

(30) Priority: 11.12.2023 CN 202311688769
(71) Applicant: Honor Device Co., Ltd., Shenzhen, Guangdong 518040 (CN)
(72) Inventor: XIE, Zhibo, Shenzhen, Guangdong 518040 (CN)
(74) Representative: Isarpatent
(86) International application number: PCT/CN2024/106552
(87) International publication number: WO 2025/123672

(57) **Abstract**

This application provides a data processing method, an electronic device, a wearable device, and a communication system, and relates to an electronic technology, to monitor a physiological parameter of a user in real time, and prompt, when it is detected that the physiological parameter is abnormal, the user to cooperate in input of related data and perform related detection. The method is applied to an electronic device, and the electronic device establishes a connection to a wearable device. The method includes: when the wearable device is worn on a body part of a user, obtaining, based on the connection, a first parameter value that corresponds to a preset physiological parameter of the user and that is collected by the wearable device, where the preset physiological parameter includes at least one of the following: a pulse wave velocity PWV, a heart rate, heart rate variability HRV, blood pressure, and a respiratory rate; analyzing the first parameter value to obtain a first analysis result; and if the first analysis result represents that the preset physiological parameter of the user is abnormal, giving first prompt information, where the first prompt information is used to prompt the user to cooperate in input of preset data, so that new analysis is performed on the preset physiological parameter.

## Description

This application claims priority to Chinese Patent Application No. 202311688769.2, filed with the China National Intellectual Property Administration on December 11, 2023 and entitled "DATA PROCESSING METHOD, ELECTRONIC DEVICE, WEARABLE DEVICE, AND COMMUNICATION SYSTEM", which is incorporated herein by reference in its entirety.

### TECHNICAL FIELD

Embodiments of this application relate to electronic technologies, and in particular, to a data processing method, an electronic device, a wearable device, and a communication system.

### BACKGROUND

Coronary atherosclerosis heart disease (briefly referred to as coronary heart disease below) is one of diseases with highest mortality rates in the world. In an actual scenario, using an electrocardiogram for initial screening on the coronary heart disease is a common method. However, the electrocardiogram usually shows a characteristic manifestation only in an attack of the coronary heart disease.

In the attack of the coronary heart disease, pain is usually regular and mild, and is usually relieved spontaneously after rest. Consequently, the pain caused by the attack of the coronary heart disease is often underestimated or ignored by people, and they do not go to the hospital for a test in time, and are likely to miss a right time for electrocardiogram detection.

### SUMMARY

Embodiments of this application provide a data processing method, an electronic device, a wearable device, and a communication system, to monitor a physiological parameter of a user in real time, and prompt, when it is detected that the physiological parameter is abnormal, the user to cooperate in input of related data and perform related detection.

To achieve the foregoing objectives, the following technical solutions are used in embodiments of this application.

According to a first aspect, a data processing method is provided. The method is applied to an electronic device, and the electronic device is connected to a wearable device. The method includes the following steps.

When the wearable device is worn on a body part of a user, the electronic device may obtain, based on the connection, a first parameter value that corresponds to a preset physiological parameter and that is collected by the wearable device. The preset physiological parameter includes at least one of the following: a pulse wave velocity PWV, a heart rate, heart rate variability HRV, blood pressure, and a respiratory rate. Then, the electronic device may analyze the first parameter value to obtain a first analysis result. If the first analysis result represents that the preset physiological parameter of the user is abnormal, first prompt information may be given. The first prompt information is used to prompt the user to cooperate in input of preset data, so that new analysis is performed on the preset physiological parameter.

In this solution, the electronic device may collect, by using the wearable device, a parameter value corresponding to the preset physiological parameter of the user, and analyze the parameter value corresponding to the preset physiological parameter. Further, if it is determined through analysis that the corresponding parameter value is abnormal, the electronic device may give the first prompt information to prompt the user to cooperate in input of the preset data, thereby facilitating further analysis on the parameter value. In this way, the wearable device is used to monitor the preset physiological parameter of the user, so that an appropriate time can be found through parameter analysis to prompt the user to pay attention to a body status and cooperate in input of related data, and a further and deeper test can be carried out. This reduces a probability that the user misses a right time for related detection due to ignoring or habituation of pain.

In a possible implementation of the first aspect, giving the first prompt information may specifically include: The electronic device gives the first prompt information; and/or the electronic device sends notification information to the wearable device, so that the wearable device gives the first prompt information. This can better prompt the user to pay attention to a body status in time.

In a possible implementation of the first aspect, the first prompt information may be specifically given in different prompt forms such as sound, vibration, indicator light, and/or image. This enriches a prompt form.

In a possible implementation of the first aspect, the preset data includes sense information of a target body part of the user. After the first prompt information is given, the method further includes: obtaining the sense information of the target body part input by the user. Then, the electronic device may further analyze the sense information of the target body part and the first parameter value to obtain a second analysis result, so as to determine whether an abnormality of the preset physiological parameter is associated with the target body part. If the abnormality of the preset physiological parameter is associated with the target body part, it indicates that the target body part may be abnormal. In addition, if the second analysis result represents that the target body part of the user is abnormal, the electronic device may give second prompt information. The second prompt information is used to prompt the user to perform preset detection by using the wearable device.

In this solution, if the first parameter value is abnormal, the electronic device may prompt the user to input the sense information of the target body part, for example, whether a pain symptom occurs and a characteristic manifestation during pain. After receiving the sense information of the target body part input by the user, the electronic device may further analyze, with reference to the sense information, whether an abnormality of the detected first parameter value is associated with the target body part. Finally, if it is determined that the second analysis result represents that the target body part is abnormal, the electronic device may prompt the user to perform preset detection. In this way, if the target body part may be abnormal, the user may be prompted in time to perform related detection, to avoid missing a right time for detection.

In addition, after the preset detection is performed by using the wearable device, a detection result of the preset detection in a period in which an abnormality may occur may be stored. This can subsequently help the user with further detection or diagnosis.

In a possible implementation of the first aspect, the target body part may be a chest. In this way, the electronic device may analyze whether the abnormality of the preset physiological parameter is associated with a body part near the chest. This helps the user better understand a body status.

In a possible implementation of the first aspect, analyzing the sense information of the target body part and the first parameter value to obtain the second analysis result may specifically include: inputting the sense information of the target body part and the first parameter value into a first preset prompt model. The first preset prompt model is determined through training. The electronic device may obtain a first output result of the first preset prompt model. The second analysis result includes the first output result, and the first output result is used to represent a probability that the target body part of the user is abnormal. In this solution, giving the second prompt information if the second analysis result represents that the target body part of the user is abnormal may specifically include: if the first output result is greater than a first preset threshold and is less than or equal to a second preset threshold, giving the second prompt information. The second preset threshold is greater than the first preset threshold. Both the first preset threshold and the second preset threshold may be determined according to the first preset prompt model.

In this solution, the preset prompt model predetermined through training is used, so that the probability that the target body part is abnormal can be quickly determined based on the first parameter value and the sense information of the target body part. In addition, the output probability is compared with the two preset thresholds determined according to the preset prompt model. If the probability is greater than the first preset threshold and is less than or equal to the second preset threshold, the second prompt information is used to inform the user that the target body part is abnormal.

In a possible implementation of the first aspect, the method may further include: if the first output result is greater than the second preset threshold, giving third prompt information. The third prompt information is used to indicate that the target body part of the user is abnormal, and an urgency degree corresponding to the third prompt information is higher than an urgency degree corresponding to the second prompt information.

In a possible implementation of the first aspect, both the second prompt information and the third prompt information are in a sound prompt form. That an urgency degree corresponding to the third prompt information is higher than an urgency degree corresponding to the second prompt information may be specifically manifested in that prompt duration of the third prompt information is longer than prompt duration of the second prompt information, or may be manifested in that prompt volume of the third prompt information is higher than prompt volume of the second prompt information, or may be manifested in that prompt sound of the third prompt information is more urgent than prompt sound of the second prompt information. In this way, an urgency degree can be indicated to the user more intuitively.

In a possible implementation of the first aspect, both the second prompt information and the third prompt information are in a vibration prompt form. That an urgency degree corresponding to the third prompt information is higher than an urgency degree corresponding to the second prompt information may be specifically manifested in that vibration duration of the third prompt information is longer than vibration duration of the second prompt information, or may be manifested in that a vibration degree of the third prompt information is greater than a vibration degree of the second prompt information. In this way, an urgency degree can be indicated to the user more intuitively.

In a possible implementation of the first aspect, both the second prompt information and the third prompt information are in an indicator light prompt form. That an urgency degree corresponding to the third prompt information is higher than an urgency degree corresponding to the second prompt information may be specifically manifested in that an indicator light blinking frequency of the third prompt information is greater than an indicator light blinking frequency of the second prompt information, or may be manifested in that an indicator light color of the third prompt information is brighter than an indicator light color of the second prompt information. In this way, an urgency degree can be indicated to the user more intuitively.

In a possible implementation of the first aspect, analyzing the first parameter value to obtain the first analysis result may specifically include: inputting the first parameter value into a second preset prompt model, where the second preset prompt model is determined through training; and obtaining a second output result of the second preset prompt model, where the first analysis result includes the second output result, and the second output result is used to represent a probability that the preset physiological parameter of the user is abnormal. In this solution, that the first analysis result represents that the preset physiological parameter of the user is abnormal includes: The second output result is greater than a third preset threshold.

In this solution, the preset prompt model predetermined through training is used, so that the probability that the preset physiological parameter is abnormal can be quickly determined based on the first parameter value. In addition, whether the first parameter value is abnormal can also be quickly determined by comparing the output probability with the preset threshold determined according to the preset prompt model.

In a possible implementation of the first aspect, the electronic device stores a reference value corresponding to the preset physiological parameter and preset user information. Analyzing the first parameter value to obtain the first analysis result may specifically include: analyzing the reference value, the preset user information, and the first parameter value to obtain the first analysis result. In this way, whether the first parameter value is abnormal can be determined with reference to the reference value and the preset user information, so that the analysis result is more accurate.

In a possible implementation of the first aspect, the analyzing the reference value, the preset user information, and the first parameter value to obtain the first analysis result may specifically include: inputting the reference value, the preset user information, and the first parameter value into the second preset prompt model, and obtaining the second output result of the second preset prompt model.

In a possible implementation of the first aspect, different users have different ages, genders, and habits, and may have different preset physiological parameters. Therefore, the analyzing the reference value, the preset user information, and the first parameter value to obtain the first analysis result may specifically include: with reference to the reference value and the preset user information, determining a normal indicator range corresponding to the preset physiological parameter. Then, the electronic device determines whether the first parameter value corresponding to the preset physiological parameter is out of the corresponding normal indicator range. In this solution, that the first analysis result represents that the preset physiological parameter of the user is abnormal may specifically include: first parameter values corresponding to at least a preset quantity of preset physiological parameters are out of corresponding normal indicator ranges. The preset quantity may be set according to a time and a condition, for example, set to 2 or 3.

In a possible implementation of the first aspect, preset physiological parameters each correspond to a different value output frequency. Analyzing the first parameter value to obtain the first analysis result may specifically include: analyzing first parameter values obtained in a same cycle to obtain the first analysis result. The electronic device obtains, in the same cycle, parameter values corresponding to the preset physiological parameters. In other words, in one cycle, the electronic device may obtain parameter values corresponding to the preset physiological parameters, but value output frequencies corresponding to different preset physiological parameters are different. In this solution, the method may further include: when it is detected that a first parameter value corresponding to a first preset physiological parameter is out of a corresponding normal indicator range, immediately obtaining a third parameter value corresponding to a second preset physiological parameter by using the wearable device. The first preset physiological parameter includes one or more of the preset physiological parameters, and the second preset physiological parameter includes a physiological parameter other than the first preset physiological parameter. In other words, in one cycle, if parameter values corresponding to some preset physiological parameters first obtained by the electronic device are out of corresponding normal indicator ranges, the wearable device may be triggered to immediately collect a parameter value corresponding to another preset physiological parameter, and there is no need to wait until a value output moment for another preset physiological parameter in the cycle. Then, the electronic device may analyze the reference value, the preset user information, the first parameter value corresponding to the first preset physiological parameter, and the third parameter value to obtain a third analysis result. In addition, if the third analysis result represents that the preset physiological parameter of the user is abnormal, the electronic device may also give the first prompt information. This can accelerate collection of physiological parameters when it is determined that the user may be abnormal based on some physiological parameters. Therefore, the user is prompted more quickly when an abnormality occurs to the user.

In a possible implementation of the first aspect, after electrocardiogram detection prompt information is given, the method further includes: in response to an operation of enabling an electrocardiogram detection function, performing single-lead electrocardiogram detection; obtaining a first detection result of the single-lead electrocardiogram detection; and if the first detection result is normal, performing multi-lead electrocardiogram detection.

In a possible implementation of the first aspect, the performing multi-lead electrocardiogram detection may specifically include: first performing first multi-lead electrocardiogram detection, and obtaining a second detection result of the first multi-lead electrocardiogram detection. Then, if the second detection result is normal, second multi-lead electrocardiogram detection is performed. Next, a third detection result of the second multi-lead electrocardiogram detection is obtained. Finally, if the third detection result is normal, it indicates that the user is normal. In this case, the electronic device may give health prompt information. The multi-lead electrocardiogram detection includes the first multi-lead electrocardiogram detection and the second multi-lead electrocardiogram detection. In this way, the user may be sequentially prompted to perform electrocardiogram detection.

In a possible implementation of the first aspect, the method further includes: if the first detection result is abnormal, or the second detection result is abnormal, or the third detection result is abnormal, giving fourth prompt information. The fourth prompt information is used to indicate that the detection result of the electrocardiogram detection is abnormal, and an urgency degree corresponding to the fourth prompt information is higher than an urgency degree corresponding to the first prompt information. A posture used by the user to cooperate in the multi-lead electrocardiogram detection is more complicated than a posture used by the user to cooperate in the single-lead electrocardiogram detection. Therefore, the single-lead electrocardiogram detection is before the multi-lead electrocardiogram detection. If the result of the single-lead electrocardiogram detection is normal, the user may be prompted to pay attention to a body status. The user may choose to perform the single-lead electrocardiogram detection again, or choose whether to perform the multi-lead electrocardiogram detection. This can provide better comfort for the user.

In a possible implementation of the first aspect, the electronic device stores a reference value corresponding to the preset physiological parameter and preset user information. Analyzing the sense information of the target body part and the first parameter value to obtain the second analysis result may specifically include: analyzing the reference value, the preset user information, the sense information of the target body part, and the first parameter value to obtain the second analysis result. In this way, whether the first parameter value is abnormal can be determined with reference to the reference value and the preset user information, so that the analysis result is more accurate.

In a possible implementation of the first aspect, the analyzing the reference value, the preset user information, the sense information of the target body part, and the first parameter value to obtain the second analysis result may specifically include: inputting the reference value, the preset user information, the sense information of the target body part, and the first parameter value into the first preset prompt model. The first preset prompt model is determined through training. The electronic device may obtain the first output result of the first preset prompt model. The second analysis result includes the first output result, and the first output result is used to represent the probability that the target body part of the user is abnormal. In this solution, giving the second prompt information if the second analysis result represents that the target body part of the user is abnormal may specifically include: if the first output result is greater than the first preset threshold and is less than or equal to the second preset threshold, giving the second prompt information. The second preset threshold is greater than the first preset threshold. Both the first preset threshold and the second preset threshold may be determined according to the first preset prompt model.

In a possible implementation of the first aspect, before the first parameter value corresponding to the preset physiological parameter of the user is obtained, the method further includes: displaying a first interface, where the first interface is used to input preset user information. Then, preset user information input by the user in the first interface is received and saved. The preset user information may include age and gender of the user.

In a possible implementation of the first aspect, the preset user information may further include one or more of the following: whether the user has a history of chest pain (a typical type of chest pain of coronary heart disease), whether the user has diabetes, whether the user has hypertension, whether the user has dyslipidemia, and whether the user smokes.

In a possible implementation of the first aspect, the first interface further includes a first control, and the first control is used to trigger collection of the reference value of the preset physiological parameter. In response to a triggering operation performed on the first control, the electronic device may send to the wearable device, and obtain a second parameter value corresponding to the preset physiological parameter by using the wearable device. The second parameter value is saved as the reference value of the preset physiological parameter.

In a possible implementation of the first aspect, the electronic device may display the first interface at a specific time interval, to prompt the user to input preset user information and a reference value of the preset physiological parameter again. In this way, the data can be updated and closer to an actual situation of the user.

In a possible implementation of the first aspect, the preset physiological parameter includes the PWV, and the corresponding first parameter value may include a PWV value.

In a possible implementation of the first aspect, the preset physiological parameter includes the heart rate, and the corresponding first parameter value may include parameter values such as a heart rate value, a heart rate increase degree, and a heart rate decrease rate.

In a possible implementation of the first aspect, the preset physiological parameter includes the HRV, and the corresponding first parameter value may include related parameters of the HRV, for example, a standard deviation of normal-to-normal intervals and a proportion of successive normal-to-normal intervals that differ by more than 50 ms.

In a possible implementation of the first aspect, the preset physiological parameter includes the blood pressure, and the corresponding first parameter value may include parameter values such as a blood pressure value, a blood pressure increase degree, and a blood pressure decrease ratio per minute.

In a possible implementation of the first aspect, the preset physiological parameter includes the respiratory rate, and the corresponding first parameter value may include a respiratory rate value.

According to a second aspect, this application provides a data processing method. The method is applied to a wearable device, and the wearable device establishes a connection to an electronic device. The method includes: when the wearable device is worn on a body part of a user, collecting a first parameter value corresponding to a preset physiological parameter of the user; and sending the first parameter value to the electronic device through the connection. The first parameter value is to be analyzed by the electronic device to obtain a first analysis result. If the first analysis result represents that the preset physiological parameter of the user is abnormal, first prompt information is given, where the first prompt information is used to prompt the user to cooperate in input of preset data, so that new analysis is performed on the preset physiological parameter.

In a possible implementation of the second aspect, the wearable device may collect a parameter value corresponding to the preset physiological parameter in real time.

According to a third aspect, this application provides a data processing method. The method is applied to a wearable device. The method includes: when the wearable device is worn on a body part of a user, collecting a first parameter value corresponding to a preset physiological parameter of the user, where the preset physiological parameter includes at least one of the following: a pulse wave velocity PWV, a heart rate, heart rate variability HRV, blood pressure, and a respiratory rate; obtaining a reference value corresponding to the preset physiological parameter and preset user information; analyzing the first parameter value to obtain a first analysis result; and if the first analysis result represents that the preset physiological parameter of the user is abnormal, giving first prompt information, where the first prompt information is used to prompt the user to cooperate in input of preset data, so that new analysis is performed on the preset physiological parameter.

In this solution, the wearable device can collect and analyze a parameter value corresponding to the preset physiological parameter, and perform prompting when the parameter value is abnormal.

According to a fourth aspect, this application further provides an electronic device. The electronic device may include a processor and a memory. The memory is configured to store computer-executable instructions. When the electronic device runs, the processor executes the computer-executable instructions stored in the memory, to enable the electronic device to perform the data processing method according to any one of the first aspect or the data processing method according to any one of the third aspect.

According to a fifth aspect, this application further provides a wearable device. The wearable device may include a photoplethysmography PPG sensor, a processor, and a memory. The PPG sensor and the memory are separately coupled to the processor. The PPG sensor is configured to collect a parameter value corresponding to a preset physiological parameter. The memory stores computer program code, and the computer program code includes computer instructions. The processor executes the computer-executable instructions stored in the memory, to enable the wearable device to perform the data processing method according to any one of the second aspect.

In a possible implementation of the fifth aspect, the wearable device may further include an electrocardiogram ECG sensor. The ECG sensor is coupled to the processor. The ECG sensor is configured to perform electrocardiogram detection.

According to a sixth aspect, this application further provides a communication system. The communication system includes the electronic device according to any one of the fourth aspect and the wearable device according to any one of the fifth aspect. The electronic device establishes a connection to the wearable device, and the electronic device may obtain, based on the connection, a parameter value that corresponds to a preset physiological parameter and that is collected by the wearable device.

According to a seventh aspect, this application provides a computer-readable storage medium. The computer-readable storage medium stores instructions. When the instructions are run on a computer, the computer is enabled to perform the data processing method according to any one of the first aspect.

According to an eighth aspect, a computer program product including instructions is provided. When the computer program product is run on an electronic device, the electronic device is enabled to perform the data processing method according to any one of the first aspect.

According to a ninth aspect, an apparatus (for example, the apparatus may be a chip system) is provided. The apparatus includes a processor, configured to support an electronic device in implementing a function involved in the first aspect. In a possible design, the apparatus further includes a memory. The memory is configured to store necessary program instructions and data of the electronic device. When the apparatus is a chip system, the apparatus may include a chip, or may include a chip and another discrete device.

For technical effects of any one of the designs in the second aspect to the ninth aspect, refer to the technical effects of the designs in the first aspect. Details are not described herein again.

### BRIEF DESCRIPTION OF DRAWINGS

FIG. 1 is a schematic diagram of a structure of a communication system according to an embodiment of this application;
FIG. 2 is a schematic diagram of a hardware structure of an electronic device according to an embodiment of this application;
FIG. 3 is a schematic diagram of a hardware structure of a wearable device according to an embodiment of this application;
FIG. 4 is a schematic diagram of a structure of a wearable device according to an embodiment of this application;
FIG. 5 is a schematic diagram of a structure of a wearable device according to an embodiment of this application;
FIG. 6 is a schematic diagram of an ECG of 12 leads according to an embodiment of this application;
FIG. 7 is a schematic diagram of ECGs of six limb leads according to an embodiment of this application;
FIG. 8 is a schematic diagram of a posture used in detection of six limb leads according to an embodiment of this application;
FIG. 9 is a comparison diagram of six preset positions and six equivalent positions on the front of a chest according to an embodiment of this application;
FIG. 10 is a diagram of a detection principle of equivalent precordial leads according to an embodiment of this application;
FIG. 11 is a schematic diagram of a posture and a detection electrode position that are used in detection of six precordial leads according to an embodiment of this application;
FIG. 12 is a schematic flowchart of a data processing method according to an embodiment of this application;
FIG. 13A and FIG. 13B are a schematic flowchart of a data processing method according to an embodiment of this application;
FIG. 14A to FIG. 14D are schematic diagrams of an interface of a mobile phone according to an embodiment of this application;
FIG. 15A to FIG. 15C are schematic diagrams of an interface of a mobile phone according to an embodiment of this application;
FIG. 16 is a schematic diagram of a process in which a smart watch interacts with a mobile phone according to an embodiment of this application;
FIG. 17 is a diagram of a principle of measuring a PWV based on parameters collected by a single-lead ECG sensor and a PPG sensor according to an embodiment of this application;
FIG. 18 is a diagram of a principle of measuring a PWV based on parameters collected by a single-lead ECG sensor and a PPG sensor according to an embodiment of this application;
FIG. 19A and FIG. 19B are a schematic diagram of a complete procedure of a data processing method according to an embodiment of this application;
FIG. 20A to FIG. 20D are schematic diagrams of an interface of a mobile phone in a process of performing single-lead electrocardiogram detection by using a smart watch according to an embodiment of this application;
FIG. 21A to FIG. 21D are schematic diagrams of an interface of a mobile phone in a process of performing multi-lead electrocardiogram detection by using a smart watch according to an embodiment of this application;
FIG. 22A to FIG. 22D are schematic diagrams of an interface of a mobile phone in a process of performing multi-lead electrocardiogram detection by using a smart watch according to an embodiment of this application;
FIG. 23 is a schematic diagram of an interface of a smart watch according to an embodiment of this application; and
FIG. 24 is a diagram of an architecture of a chip system according to an embodiment of this application.

### DESCRIPTION OF EMBODIMENTS

For ease of understanding, the following briefly describes technical terms that may be involved in embodiments of this application.
1. Coronary heart disease can be divided into chronic coronary syndrome (chronic coronary syndrome) and acute coronary syndrome (acute coronary syndrome).
2. A pulse wave velocity (pulse wave velocity, PWV) reflects elasticity of a main artery of a human body. When the arterial elasticity is good, the pulse wave velocity is slow, and when the arterial elasticity is poor, the pulse wave velocity is fast.
3. A heart rate is a quantity of times a human heart beats per minute, and is one of basic vital signs of a human.
4. Heart rate variability (heart rate variability, HRV) reflects a status of a human autonomic nervous system. The autonomic nervous system is mainly composed of a sympathetic nervous system and a parasympathetic nervous system. Both the sympathetic nervous system and the parasympathetic nervous system control a frequency and a rhythm of heartbeat. When the sympathetic nervous system is excited, the frequency of the heartbeat increases, the rhythm is more regular, and a related parameter of the HRV changes accordingly. When a vagus nervous system in the parasympathetic nervous system is excited, the frequency of the heartbeat decreases, the rhythm becomes irregular, and the related parameter of the HRV also changes accordingly.
5. Blood pressure is lateral pressure exerted on a blood vessel wall per unit area when blood flows in a blood vessel. What is measured is generally arterial blood pressure in systemic circulation, including systolic blood pressure and diastolic blood pressure.
6. A respiratory rate is a quantity of breaths per minute. People with the coronary heart disease often experience an increased respiratory rate when the disease attacks.
7. A photoplethysmography (photoplethysmography, PPG) sensor is based on a light emitting diode (light emitting diode, LED) light source and a photodetector, measures attenuated light after reflection and absorption by a blood vessel and a tissue of a human body, records a status of pulsation of a blood vessel, and is used to monitor a heart rate, a respiratory rate, a pulse wave velocity, blood pressure, HRV, and other information based on a frequency, a rhythm, a waveform, and the like of the pulsation.

A gold standard for diagnosing the coronary heart disease is coronary angiography, but this technology is an invasive procedure and is costly. In an actual scenario, using an electrocardiogram for initial screening on the coronary heart disease is a common method. However, typical electrocardiogram manifestations are only seen in the acute coronary syndrome and an acute attack of the chronic coronary syndrome. In addition, most of the people with the coronary heart disease are people with the chronic coronary syndrome. In the absence of an acute attack, it is difficult to obtain a diagnostically valuable result through an electrocardiogram test.

Acute attack time of the chronic coronary syndrome is usually short, lasting approximately 5 to 10 minutes. Consequently, even if people with the chronic coronary syndrome go to the hospital for a test after an acute attack, it is difficult to obtain a clear diagnosis because attack time has been missed. A commonly-used screening method in hospitals is a treadmill exercise test, which increases exercise load of a patient to induce an acute attack of the coronary heart disease and continuously monitors an electrocardiogram during exercise to obtain a clear diagnosis. However, this test takes a lot of time and has specific risks. In addition, in an attack of the chronic coronary syndrome, pain is usually regular and mild, and is usually relieved spontaneously after rest. Consequently, the pain caused by the attack of the coronary heart disease is often underestimated or ignored by people, and they do not go to the hospital for a test in time, which eventually leads to worsening of the disease.

The acute coronary syndrome often occurs rapidly and is severe, with a characteristic change in a related physiological indicator and a characteristic manifestation of an electrocardiogram. However, most of people with the acute coronary syndrome develop from the chronic coronary syndrome. Habituation and underestimation of an attack of chest pain may delay treatment, resulting in failure to receive timely diagnosis and treatment, which is life-threatening.

In a related technology, a multi-lead electrocardiogram (electrocardiogram, ECG) sensor is disposed in an electronic device (for example, a wearable device), and the electronic device can detect and record an electrocardiosignal of a user by using the multi-lead ECG sensor. However, in some cases, the user may ignore a symptom such as chest pain that the user experiences, or if there is no obvious chest pain in an attack of the coronary heart disease, the user may not be able to accurately find a right time to use the multi-lead ECG sensor for electrocardiogram detection.

In view of this, embodiments of this application provide a data processing method and an electronic device. In this method, a parameter value of a physiological parameter may be obtained in real time by using a wearable device connected to an electronic device. Therefore, a change in a physiological parameter of a user may be monitored in real time, and when it is detected that the physiological parameter satisfies a specific condition, the user is prompted to cooperate in input of preset data, so as to further analyze a parameter value of the physiological parameter. In this way, the wearable device is used to monitor a preset physiological parameter of the user, so that an appropriate time can be found through parameter analysis to prompt the user to pay attention to a body status and cooperate in input of related data, and a further and deeper test can be carried out. This reduces a probability that the user misses a right time for related detection due to ignoring or habituation of pain.

People with the coronary heart disease may suffer from an attack of the coronary heart disease under some inducing factors. In this case, they may have a related symptom (chest pain) and changes in some indicators, such as a PWV, HRV, a heart rate, blood pressure, a respiratory rate, heart rate recovery, and blood pressure recovery. A most typical characteristic of an acute attack of the coronary heart disease is a change in an electrocardiogram. In the data processing method proposed in embodiment of this application, changes in physiological parameters such as a PWV, HRV, a heart rate, blood pressure, and a respiratory rate are monitored in real time by using a PPG sensor. In addition, when it is detected that a change in a physiological parameter satisfies a specific condition, a user may be prompted to perform electrocardiogram detection.

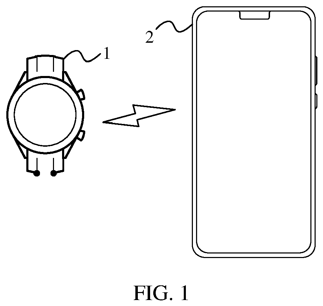

In some embodiments, the foregoing data processing method may be applied to an electronic device. The electronic device may be connected to and communicate with a wearable device. As shown in FIG. 1, an electronic device 2 is connected to and communicates with a wearable device 1. The wearable device is usually worn on a body part, such as a wrist, of a user for a long time. A PPG sensor may be disposed on the wearable device. When the user wears the wearable device on a body part, the wearable device may monitor changes in physiological parameters such as a PWV, HRV, a heart rate, blood pressure, and a respiratory rate by using the PPG sensor in real time. Then, the electronic device obtains the foregoing physiological parameter of the user from the wearable device. Next, the electronic device may analyze a change in the physiological parameter of the user, and perform prompting based on an analysis result.

For example, the electronic device may be a mobile phone, a tablet computer, a personal computer (personal computer, PC), a smart screen, a desktop computer, a laptop computer, a handheld computer, a notebook computer, an ultra-mobile personal computer (ultra-mobile personal computer, UMPC), a netbook, a wearable device such as a smart watch, an artificial intelligence (artificial intelligence, AI) sound box, or a vehicle-mounted device, or may be various teaching aids (for example, a learning machine and an early education machine), a smart toy, a portable robot, a personal digital assistant (personal digital assistant, PDA), an augmented reality (augmented reality, AR)/virtual reality (virtual reality, VR) device, a media player, or the like, or may be a device with a mobile office function, a device with a smart home function, a device with audio and video entertainment functions, a device that supports smart travel, or the like. A specific form of the device is not specially limited in embodiments of this application.

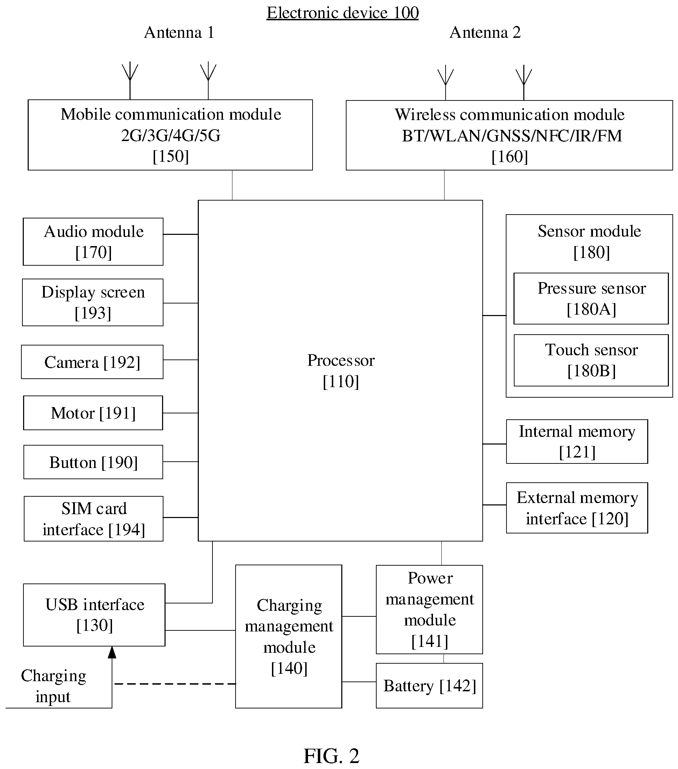

FIG. 2 shows a hardware structure of an electronic device 100 according to some embodiments. The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a sensor module 180, a button 190, a motor 191, a camera 192, a display screen 193, a subscriber identity module (subscriber identification module, SIM) card interface 194, and the like. The sensor module 180 may include a pressure sensor 180A, a touch sensor 180B, and the like.

It can be understood that the structure shown in embodiments of this application does not constitute a specific limitation on the electronic device 100. In some other embodiments of this application, the electronic device 100 may include more or fewer components than those shown in the figure, or combine some components, or split some components, or have a different component arrangement. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), and/or the like. Different processing units may be separate devices, or may be integrated into one or more processors. For example, the processor 110 is configured to perform the data processing method in embodiments of this application.

The controller may be a neural center and a command center of the electronic device 100. The controller may generate an operation control signal based on instruction operation code and a time sequence signal, to complete control of instruction fetching and instruction execution.

A memory may be further disposed in the processor 110, and is configured to store instructions and data. In some embodiments, the memory in the processor 110 is a cache memory. The memory may store instructions or data that has just been used or used repeatedly by the processor 110. If the processor 110 needs to use the instructions or the data again, the processor may directly invoke the instructions or the data from the memory. This avoids repeated access and reduces waiting time of the processor 110, thereby improving system efficiency.

The USB interface 130 is an interface that complies with a USB standard specification. The USB interface 130 may be configured to connect to a charger to charge the electronic device 100, or may be used for data transmission between the electronic device 100 and a peripheral device.

The external memory interface 120 may be configured to connect to an external memory card, for example, a micro SD card, to extend a storage capability of the electronic device 100. The internal memory 121 may be configured to store computer-executable program code, and the executable program code includes instructions.

The charging management module 140 is configured to receive a charging input from the charger. The charger may be a wireless charger, or may be a wired charger. In some embodiments of wired charging, the charging management module 140 may receive a charging input from a wired charger through the USB interface 130.

The power management module 141 is configured to connect to the battery 142, the charging management module 140, and the processor 110. The power management module 141 receives an input from the battery 142 and/or the charging management module 140 and supplies power to the processor 110, the internal memory 121, an external memory, the display screen 193, the camera 192, the wireless communication module 160, and the like.

In some other embodiments, the power management module 141 may alternatively be disposed in the processor 110. In some other embodiments, the power management module 141 and the charging management module 140 may alternatively be disposed in a same component.

A wireless communication function of the electronic device 100 may be implemented by using the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna of the electronic device 100 may be configured to cover one or more communication frequency bands. Different antennas may be multiplexed, to improve antenna utilization. For example, the antenna 1 may be multiplexed as a diversity antenna of a wireless local area network. In some other embodiments, the antenna may be used in combination with a tuning switch.

The mobile communication module 150 may provide a wireless communication solution that includes 2G/3G/4G/5G or the like and that is applied to the electronic device 100. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit a processed electromagnetic wave to the modem processor for demodulation. The mobile communication module 150 may further amplify a signal modulated by the modem processor, and convert an amplified signal into an electromagnetic wave for radiation through the antenna 1.

The wireless communication module 160 may provide a wireless communication solution that is applied to the electronic device 100 and that includes a wireless local area network (wireless local area networks, WLAN) (for example, a Wi-Fi network), Bluetooth (bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processing module. The wireless communication module 160 receives an electromagnetic wave through the antenna 2, performs frequency modulation and filtering processing on the electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, in the electronic device 100, the antenna 1 and the mobile communication module 150 are coupled, and the antenna 2 and the wireless communication module 160 are coupled, so that the electronic device 100 can communicate with a network and another device by using a wireless communication technology.

The electronic device 100 may implement an audio function, for example, music playing or recording, by using the audio module 170, the application processor, and the like.

The audio module 170 is configured to convert a digital audio signal into an analog audio signal output, and also configured to convert an analog audio input into a digital audio signal. The audio module 170 may be further configured to encode and decode audio signals. In some embodiments, the audio module 170 may be disposed in the processor 110, or some functional modules in the audio module 170 are disposed in the processor 110.

The pressure sensor 180A is configured to sense a pressure signal, and may convert the pressure signal into an electrical signal. In some embodiments, the pressure sensor 180A may be disposed on the display screen 193. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure strength based on the change in the capacitance. When a touch operation is performed on the display screen 193, the electronic device 100 detects intensity of the touch operation based on the pressure sensor 180A. The electronic device 100 may further calculate a touch position based on a detection signal of the pressure sensor 180A.

The touch sensor 180B is also referred to as a "touch panel". The touch sensor 180B may be disposed on the display screen 193. The touch sensor 180B and the display screen 193 form a touchscreen, which is also referred to as a "touch screen". The touch sensor 180B is configured to detect a touch operation on or near the touch sensor. The touch sensor may transfer the detected touch operation to the application processor to determine a type of the touch event. A visual output related to the touch operation may be provided by using the display screen 193. In some other embodiments, the touch sensor 180B may alternatively be disposed on a surface of the electronic device 100, at a position different from that of the display screen 193.

The button 190 includes a power on/off button, a volume button, and the like. The button 190 may be a mechanical button, or may be a touch button. The electronic device 100 may receive a button input, and generate a button signal input related to a user setting and function control of the electronic device 100.

The motor 191 may generate a vibration prompt. The motor 191 may be configured to provide a vibration prompt for an incoming call, and may be further configured to provide vibration feedback for a touch.

The camera 192 is configured to capture a static image or a video. In some embodiments, the electronic device 100 may include 1 or N cameras 192, where N is a positive integer greater than 1.

The electronic device 100 implements a display function by using the GPU, the display screen 193, the application processor, and the like. The GPU is a microprocessor for image processing, and is connected to the display screen 193 and the application processor. The GPU is configured to perform mathematical and geometric computing for graphics rendering. The processor 110 may include one or more GPUs that perform program instructions to generate or change display information.

The display screen 193 is configured to display an image, a video, or the like. In some embodiments, the electronic device 100 may include 1 or N display screens 193, where N is a positive integer greater than 1.

The SIM card interface 194 is configured to connect to a SIM card. The SIM card may be inserted into the SIM card interface 194 or removed from the SIM card interface 194, to be in contact with or be separated from the electronic device 100. The electronic device 100 may support 1 or N SIM card interfaces, where N is a positive integer greater than 1.

Data processing methods in the following embodiments each may be performed in an electronic device 100 that has the foregoing hardware structure.

For example, the foregoing wearable device may be specifically a portable and wearable electronic device such as a sports band, a smart watch, or a smart armband. A specific form of the wrist wearable electronic device is not specially limited in embodiments of this application.

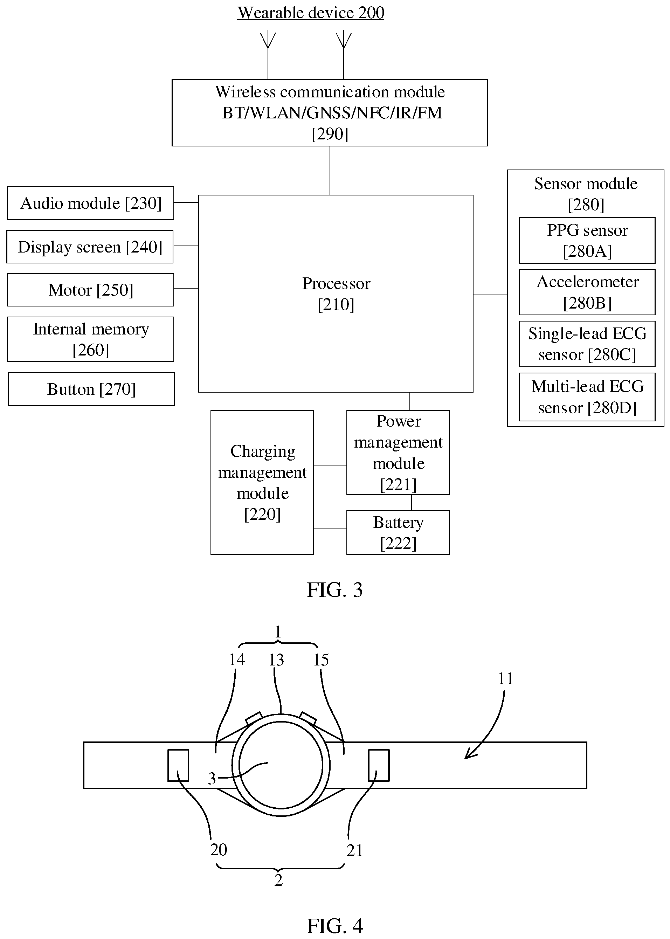

FIG. 3 shows a hardware structure of a wearable device 200 according to some embodiments. The wearable device 200 may include a processor 210, a charging management module 220, a power management module 221, a battery 222, an audio module 230, a display screen 240, a motor 250, an internal memory 260, a button 270, a sensor module 280, a wireless communication module 290, and the like. The sensor module 280 may include a PPG sensor 280A, an accelerometer 280B, a single-lead ECG sensor 280C, and a multi-lead ECG sensor 280D.

It can be understood that the structure shown in embodiments of this application does not constitute a specific limitation on the wearable device 200. In some other embodiments of this application, the wearable device 200 may include more or fewer components than those shown in the figure, or combine some components, or split some components, or have a different component arrangement. The components shown in the figure may be implemented by hardware, software, or a combination of software and hardware.

For related descriptions of the processor 210, the charging management module 220, the power management module 221, the audio module 230, the display screen 240, the motor 250, the internal memory 260, the button 270, and the wireless communication module 290, refer to descriptions of a same component in the electronic device 100 shown in FIG. 2.

The PPG sensor 280A is based on a light emitting diode (light emitting diode, LED) light source and a photodetector (photodetector, PD), measures attenuated light after reflection and absorption by a blood vessel and a tissue of a human body, records a status of pulsation of a blood vessel, and is used to monitor a heart rate, a respiratory rate, a pulse wave velocity, and other information based on a frequency, a rhythm, a waveform, and the like of the pulsation.

The accelerometer 280B is a meter for measuring a linear acceleration of a carrier. In embodiments of this application, the accelerometer 280B may be configured to detect whether a user is in an exercise state.

The single-lead ECG sensor 280C includes two electrodes, and may be configured to detect an electrocardiosignal.

The multi-lead ECG sensor 280D usually includes more than two electrodes, and may be configured to detect an electrocardiosignal. The multi-lead ECG sensor 280D is compatible with the single-lead ECG sensor 280C.

In an embodiment of this application, the wearable device uses a multi-electrode (for example, 10 electrodes) ECG sensor (that is, a multi-lead ECG sensor), to implement a detection solution of a wearable 12-lead ECG sensor. Nine electrodes are detection electrodes, and the other electrode is a drive electrode that is configured to eliminate common-mode interference.

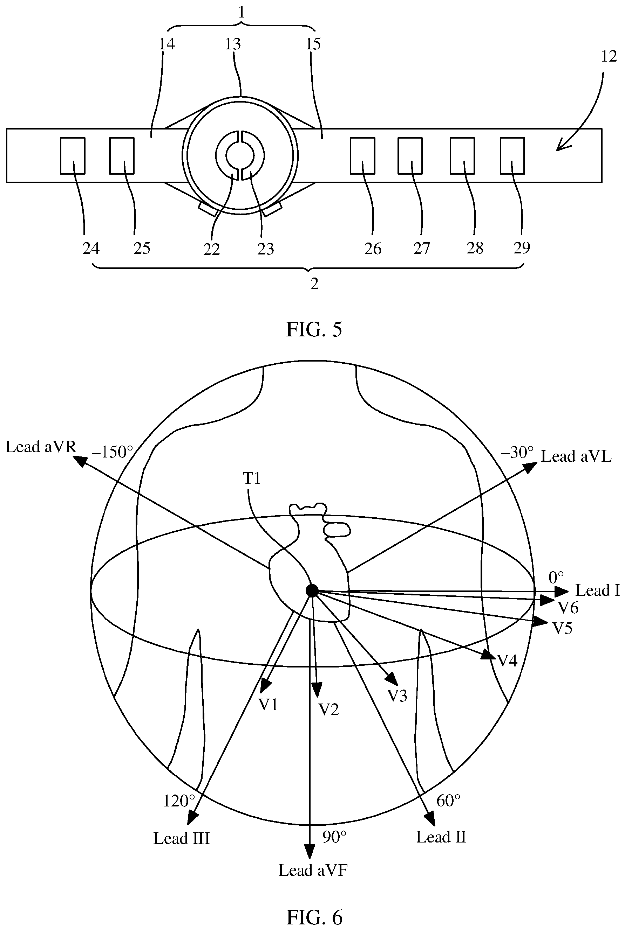

An example in which the wearable device is a wrist wearable device, for example, a smart watch, is used below for description. During measurement with a 12-lead ECG sensor, the smart watch needs to be taken off before use. As shown in FIG. 4 and FIG. 5, a smart watch 1 may include a body 13, a first fastening band 14, and a second fastening band 15. The body 13 is connected to the first fastening band 14 and the second fastening band 15.

The smart watch 1 includes a first surface 11 (that is, an outer side of the watch band) and a second surface 12 (that is, an inner side of the watch band) that are opposite to each other and that are formed by the body 13 and the two fastening bands together. A plurality of detection electrodes 2 are separately disposed on the first surface 11 and the second surface 12, and the plurality of detection electrodes 2 are configured to detect an electrical signal on a surface of a human body. Specifically, the plurality of detection electrodes 2 may include two upper limb electrodes, one lower limb electrode 23, and six precordial electrodes. The two upper limb electrodes are disposed on the first surface 11, and include a first upper limb electrode 20 and a second upper limb electrode 21 shown in FIG. 4, which are respectively RA in contact with a right upper limb and LA in contact with a left upper limb. Lower limb electrodes may be disposed on the body 13 of the smart watch on the second surface 12, and include a first lower limb electrode 22 and a second lower limb electrode 23 shown in FIG. 5, which are respectively an RL electrode in contact with a right lower limb and an LL electrode in contact with a left lower limb. The six precordial electrodes may be disposed on the first fastening band 14 and the second fastening band 15 on the second surface 12, and may include a detection electrode 24, a detection electrode 25, ..., and a detection electrode 29 shown in FIG. 5.

The smart watch 1 may be used to detect an electrocardiogram. The following describes information about an electrocardiogram. One lead in a 12-lead electrocardiogram represents a detection vector in one direction. A single detection vector can detect abnormalities in only one direction. 12 leads include six limb leads that reflect a condition of a potential of a heart in a coronal plane (a longitudinal section that divides the heart into front and back parts) and six precordial leads that reflect a condition of a potential of the heart in a transverse plane (a longitudinal section that divides the heart into upper and lower parts). The six limb leads may include a lead I, a lead II, a lead III, a lead aVL, a lead aVF, and a lead aVR shown in FIG. 6. The six precordial leads may include a lead V1, a lead V2, a lead V3, a lead V4, a lead V5, and a lead V6 shown in FIG. 6. The 12 leads may reflect electrical activity of different parts of the heart, so that comprehensive and accurate detection can be performed on the heart, for example, detection of a disease such as the coronary heart disease.

A measurement method for the six limb leads is first described below. When a user performs lead detection by using the smart watch 1, the first upper limb electrode 20 and the second upper limb electrode 21 may be in contact with a right upper limb and a left upper limb respectively, and the first lower limb electrode 22 and the second lower limb electrode 23 may be in contact with lower limbs. In this case, three detection electrodes (the first upper limb electrode 20, the second upper limb electrode 21, and the second lower limb electrode 23) can implement measurement of six limb leads in total. The first lower limb electrode 22 may be used as a drive electrode.

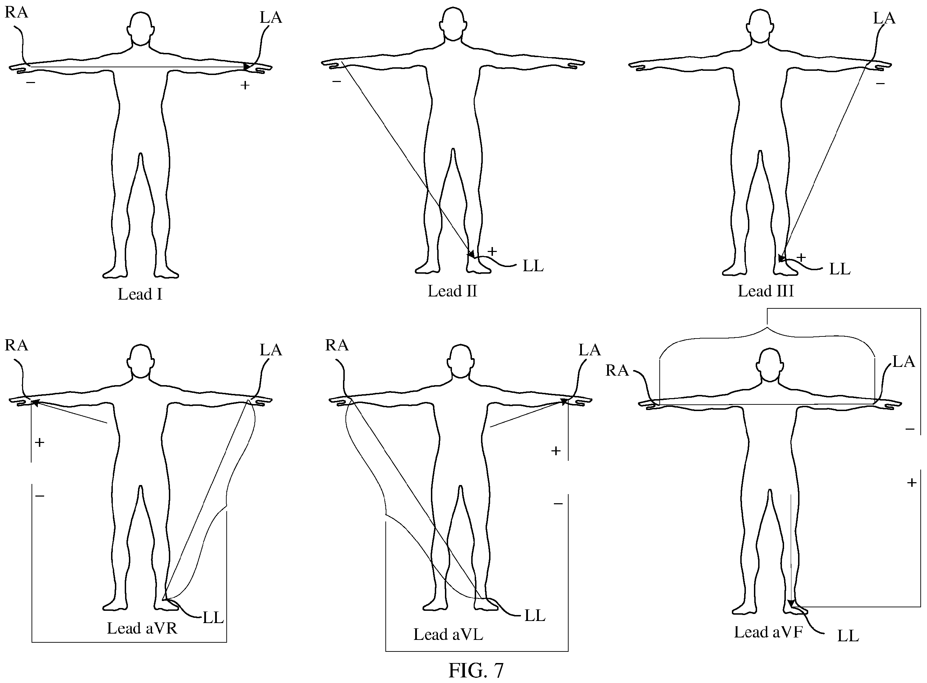

Refer to FIG. 7. A potential difference between the first upper limb electrode 20 and the second upper limb electrode 21 may be referred to as the lead I. A potential difference between the second lower limb electrode 23 and the first upper limb electrode 20 may be referred to as the lead II. A potential difference between the second lower limb electrode 23 and the second upper limb electrode 21 may be referred to as the lead III. A potential of a central terminal, namely, Wilson's central terminal, of the three detection electrodes (the first upper limb electrode 20, the second upper limb electrode 21, and the second lower limb electrode 23) may be determined by using all the three electrodes. Signals of the lead aVR, the lead aVL, and the lead aVF may be obtained by separately detecting potential differences between the central terminal and the three detection electrodes (the first upper limb electrode 20, the second upper limb electrode 21, and the second lower limb electrode 23).

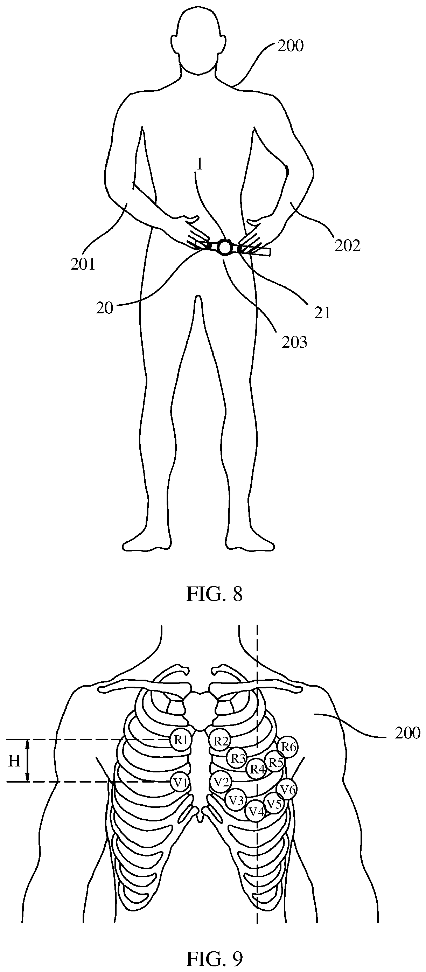

When measurement of the six limb leads is performed by using the smart watch 1 shown in FIG. 4 and FIG. 5, the smart watch needs to be taken off before use. For example, FIG. 8 is a schematic diagram of a posture used in detection of the six limb leads. When performing electrocardiogram detection of the six limb leads, the user may first place the smart watch 1 at a position on a lower limb 203 shown in FIG. 8. Specifically, a left upper limb 202 is made to be in contact with the second upper limb electrode 21, and a right upper limb 201 is made to be in contact with the first upper limb electrode 20. In addition, one side of the smart watch 1 is pressed toward a human body 200 by using the left upper limb 202 and the right upper limb 201. In this case, the second surface 12 of the smart watch 1 is in close contact with the human body 200, so that the second lower limb electrode 23 located on the second surface 12 is in full contact with the left lower limb 203. In this way, the two upper limb electrodes and the second lower limb electrode 23 can separately measure a potential of a corresponding position on the human body 200. With reference to the manner shown in FIG. 7, the six limb leads can be detected. It should be noted that the measurement manner shown in FIG. 8 shows merely an example of a posture used in measurement of the six limb leads. In another embodiment, when it is ensured that the two upper limb electrodes and the two lower limb electrodes of the smart watch are in contact with corresponding positions on the human body respectively, the user may also use another posture to measure the six limb leads.

It should be noted that the "upper limb" in embodiments of this application is a part that is below a shoulder and above a navel, and includes a hand. The "lower limb" is a part below the navel, and includes a foot, a lower abdomen, a leg, a knee, and the like. In addition, electrocardiogram measurement can be performed on both left and right lower limbs. This is not limited herein.

Next, ECG detection of the six precordial leads is described. FIG. 9 shows distribution of preset positions corresponding to the foregoing precordial leads. Six preset positions corresponding to the precordial leads on a human body may include positions V1, V2, V3, V4, V5, and V6 shown in FIG. 9. During electrocardiogram measurement, detection electrodes may be separately disposed at the six preset positions. A first preset position V1 is located at a fourth intercostal space on a right side of a sternum, a second preset position V2 is located at a fourth intercostal space on a left side of the sternum, a third preset position V3 is located at a midpoint of a connection line between the second preset position V2 and a fourth preset position V4, the fourth preset position V4 is located at an intersection of a left midclavicular line and a fifth intercostal space, a fifth preset position V5 is located on a left anterior axillary line at a same level as V4, and a sixth preset position V6 is located on a left midaxillary line at a same level as V4.

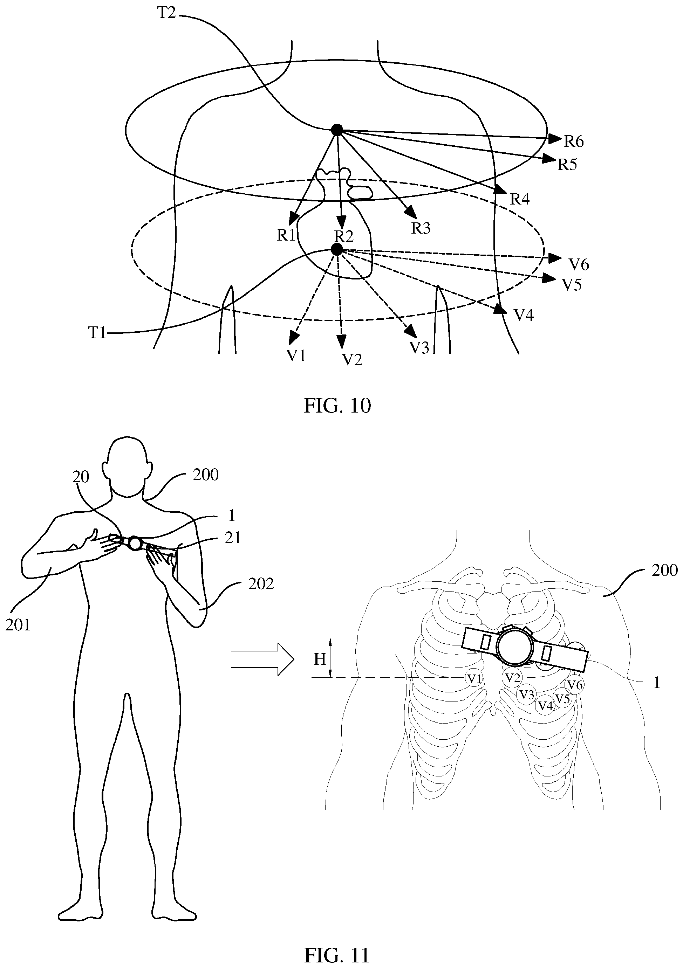

FIG. 9 further shows six equivalent positions (R1, R2, R3, R4, R5, and R6) equivalent to the foregoing six preset positions. During actual measurement, detection electrodes may be placed at the six equivalent positions, to measure an electrocardiogram of the six precordial leads. FIG. 10 is a diagram of a detection principle of equivalent precordial leads according to an embodiment of this application. In FIG. 10, vector directions pointed by a second central point T2 to the six equivalent positions R1 to R6 separately are in a one-to-one correspondence with vector directions pointed by a first central point T1 to the six preset positions V1 to V6.

When measurement of the six precordial leads is performed by using the smart watch 1 shown in FIG. 4 and FIG. 5, the smart watch needs to be taken off before use. After removing the smart watch 1 from a wrist, the user may make the second surface 12 of the smart watch 1 be in contact with the front chest of the human body. A placement position is shown in FIG. 11. The six precordial electrodes (the detection electrodes 24 to 29) of the smart watch 1 are in contact with the positions R1 to R6 shown in FIG. 9 respectively. In addition, the left upper limb is in contact with the second upper limb electrode 21, and another finger of the left upper limb presses another position on the watch band to help an electrode on the second surface 12 (that is, the inner side of the watch band) be in good contact with the body. The right upper limb is in contact with the first upper limb electrode 20, and another finger of the right upper limb presses another position on the watch band to help the precordial electrodes (the detection electrodes 24 to 29) on the second surface 12 be in good contact with the body. Based on this, differences between an upper limb central potential of the second central point T2 determined by the two upper limb electrodes and potentials of the six equivalent positions R1 to R6 on the human body measured by the six precordial electrodes may be obtained. A lead R1, a lead R2, a lead R3, a lead R4, a lead R5, and a lead R6 that are obtained may be respectively equivalent to the lead V1, the lead V2, the lead V3, the lead V4, the lead V5, and the lead V6 that correspond to vector directions of the lead R1, the lead R2, the lead R3, the lead R4, the lead R5, and the lead R6, to implement detection of the six equivalent precordial leads (the lead V1, the lead V2, the lead V3, the lead V4, the lead V5, and the lead V6). It should be noted that the measurement manner shown in FIG. 11 shows merely an example of a posture used in measurement of the six precordial leads. In another embodiment, when it is ensured that the six detection electrodes on the inner side of the watch band of the smart watch are in contact with corresponding positions on the human body respectively, the user may also use another posture to measure the six precordial leads.

The user may actively perform multi-lead electrocardiogram detection at any moment by using the smart watch 1 in the foregoing manner. However, in some cases, the user may ignore a symptom such as chest pain that the user experiences, or if there is no obvious chest pain in an attack of the coronary heart disease, the user may miss a right time for electrocardiogram detection. Therefore, an embodiment of this application provides a data processing method, which may be used to remind a user of a right time for electrocardiogram detection.

In some embodiments, when the smart watch 1 is worn on a body part (for example, a wrist) of a user, a physiological parameter value of the user may be collected in real time. Then, the smart watch 1 may transmit the collected physiological parameter value to a mobile phone (for example, a mobile phone 2) connected to the smart watch 1. The mobile phone 2 analyzes the physiological parameter value, and gives prompt information when it is detected that a change in the physiological parameter value satisfies a specific condition. It should be noted that the smart watch 1 and the mobile phone 2 may establish a connection to each other in any manner and transmit information, for example, a Bluetooth connection or a Wi-Fi connection.

In some other embodiments, the foregoing analyzing the physiological parameter value, and giving prompt information when it is detected that a change in the physiological parameter value satisfies a specific condition may alternatively be performed by the smart watch 1. That is, the smart watch 1 collects a physiological parameter value of the user in real time, and analyzes the collected physiological parameter value. Finally, when detecting that a change in the physiological parameter value satisfies a specific condition, the smart watch 1 gives prompt information to the user.

The following embodiments describe an example in which the smart watch 1 is connected to the mobile phone 2, a physiological parameter collected by the smart watch 1 in real time is transmitted to the mobile phone 2 for analysis, and prompt information is given based on an analysis result. For an implementation in which the smart watch 1 completes collection and analysis of a physiological parameter and gives prompt information, refer to implementations in embodiments of this application. Details are not described again.

In some embodiments, a function of the mobile phone 2 of obtaining a physiological parameter value from the smart watch 1, analyzing the physiological parameter value, and giving prompt information may be referred to as a physiological parameter monitoring function.

The following describes in detail a data processing method provided in embodiments of this application with reference to the accompanying drawings.

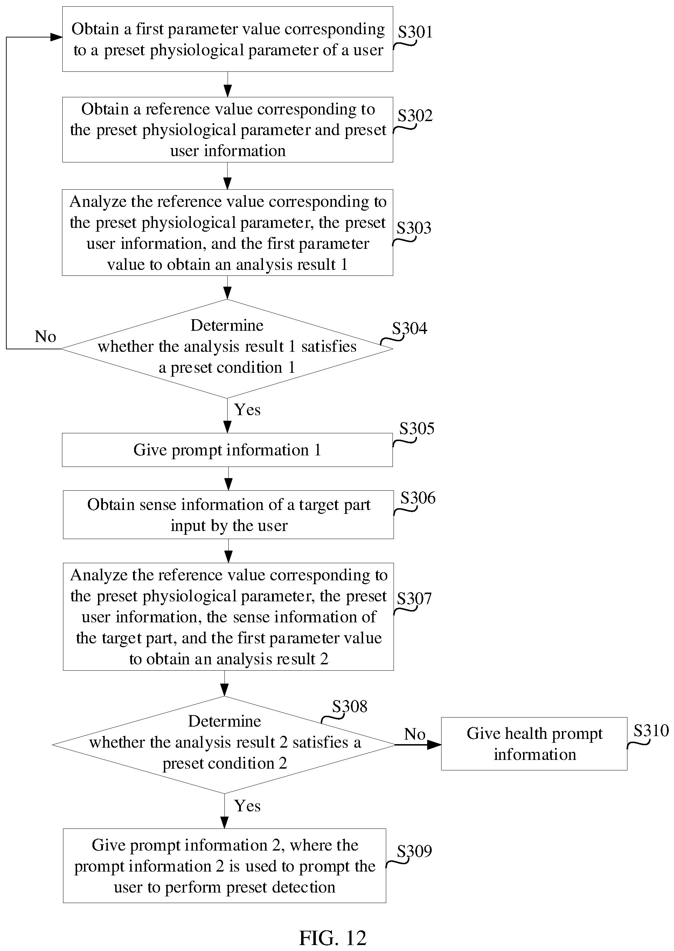

FIG. 12 shows a procedure of a data processing method according to some embodiments. The method includes the following steps.

S301: Obtain a first parameter value corresponding to a preset physiological parameter of a user.

In this embodiment of this application, the preset physiological parameter may include a physiological parameter that has a characteristic change in an attack of the coronary heart disease. In some embodiments, the preset physiological parameter may include at least one of the following: a PWV, a heart rate, HRV, blood pressure, and a respiratory rate.

When the preset physiological parameter includes the PWV, the corresponding first parameter value may include a PWV value.

When the preset physiological parameter includes the heart rate, the corresponding first parameter value may include parameter values such as a heart rate value, a heart rate increase degree, and a heart rate decrease rate.

When the preset physiological parameter includes the HRV, the corresponding first parameter value may include related parameters of the HRV, for example, a standard deviation of normal-to-normal intervals and a proportion of successive normal-to-normal intervals that differ by more than 50 ms.

When the preset physiological parameter includes the blood pressure, the corresponding first parameter value may include parameter values such as a blood pressure value, a blood pressure increase degree, and a blood pressure decrease ratio per minute.

When the preset physiological parameter includes the respiratory rate, the corresponding first parameter value may include a respiratory rate value.

It can be learned from the foregoing descriptions that when a smart watch is worn on a body part of the user, a physiological parameter of the user may be monitored in real time. Therefore, in S301, a mobile phone may obtain the first parameter value from the smart watch in real time.

When there are a plurality of preset physiological parameters, collection cycles and value output frequencies of the smart watch for the preset physiological parameters may be different. A process in which the smart watch collects parameter values of all the preset physiological parameters once is referred to as a same cycle. In other words, in one cycle, the mobile phone may obtain parameter values corresponding to the preset physiological parameters, but value output moments corresponding to different preset physiological parameters are different. In some embodiments, in S301, after the smart watch collects a parameter value corresponding to one preset physiological parameter, the mobile phone may obtain the parameter value corresponding to the preset physiological parameter from the smart watch.

In some other embodiments, in S301, the mobile phone may alternatively obtain the first parameter value from the smart watch at a specific time interval. In this way, the mobile phone may wait for the smart watch to complete collection of parameter values corresponding to all the preset physiological parameters in one cycle (one round of collection process) before the mobile phone obtains first parameter values from the smart watch. Therefore, the mobile phone may simultaneously obtain the parameter values corresponding to the preset physiological parameters.

A specific implementation process in which the smart watch collects the first parameter value corresponding to the preset physiological parameter is described in detail in a subsequent embodiment.

S302: Obtain a reference value corresponding to the preset physiological parameter and preset user information.

The reference value corresponding to the preset physiological parameter may be a parameter value that corresponds to the preset physiological parameter of the user and that is collected when the user does not suffer from a disease (for example, the coronary heart disease) attack.

In some embodiments, when the user does not suffer from an attack of the coronary heart disease and wears the smart watch on a body part, the user may actively select measurement of the preset physiological parameter on the mobile phone, and a parameter value (which may be denoted as a second parameter value) corresponding to the preset physiological parameter is collected by the smart watch. For example, the mobile phone sends a collection instruction to the smart watch in response to the operation of the user, so that the smart watch starts, in response to the collection instruction, to collect a parameter value corresponding to the preset physiological parameter. Then, the mobile phone may obtain the parameter value that corresponds to the preset physiological parameter and that is collected by the smart watch, that is, the foregoing second parameter value. Further, the mobile phone may save the parameter value corresponding to the preset physiological parameter obtained in this case as a reference value of the preset physiological parameter.

Body statuses of different users are different, normal reference ranges of physiological parameters may be different, and probabilities of the coronary heart disease are also different. The preset user information may include some body information that may be related to the coronary heart disease. In some embodiments, the preset user information may include age and gender of the user. In some other embodiments, in addition to age and gender, the preset user information may further include at least one of the following: whether the user has a history of chest pain (a typical type of chest pain of the coronary heart disease), whether the user has diabetes, whether the user has hypertension, whether the user has dyslipidemia, and whether the user smokes. The preset user information may be input by the user to the mobile phone and saved.

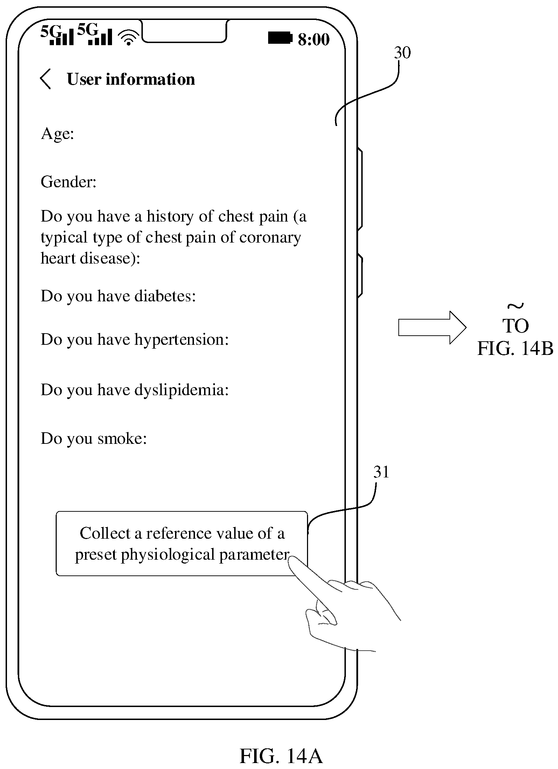

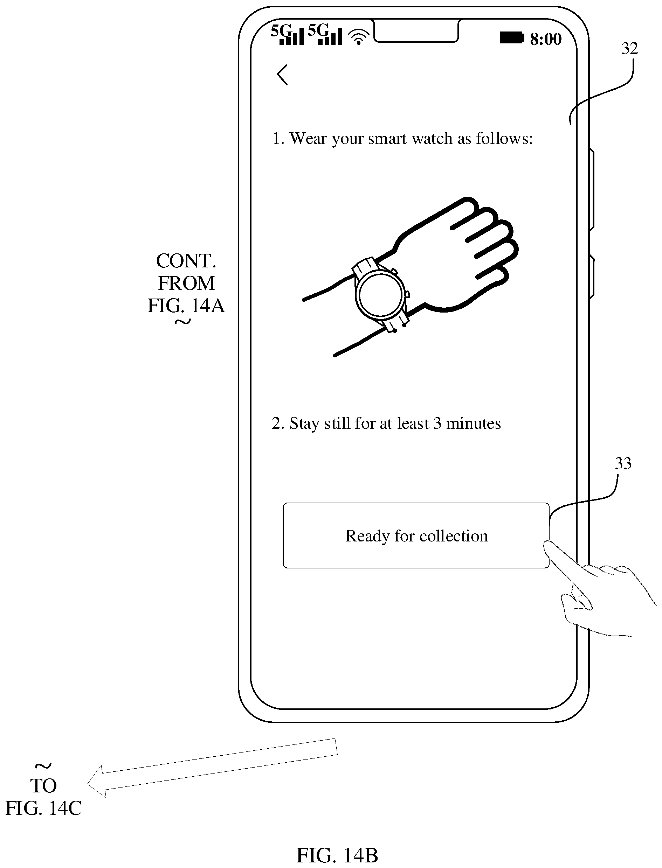

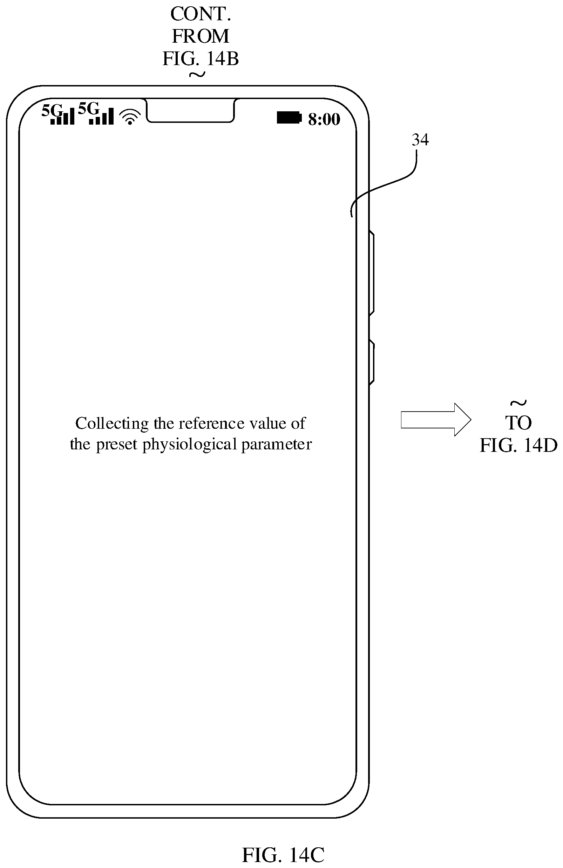

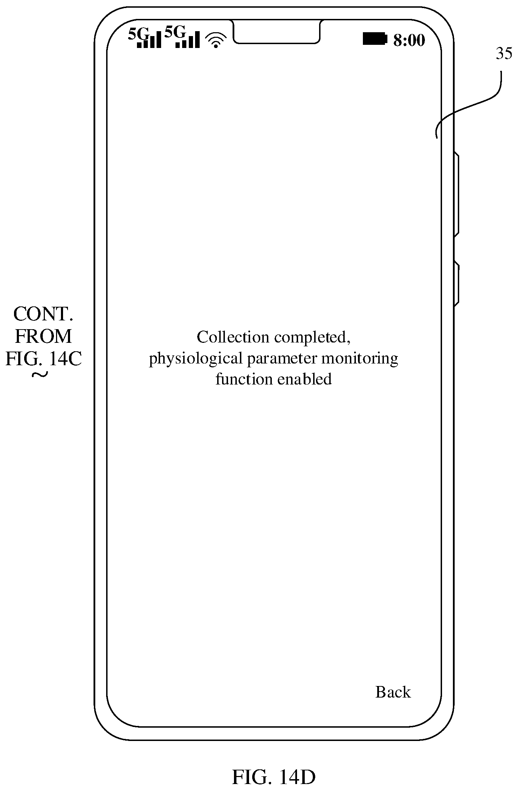

In some embodiments, when the user uses a physiological parameter monitoring function of the mobile phone for the first time, the mobile phone may prompt the user to input the preset user information, and collect the reference value corresponding to the preset physiological parameter. After that, the mobile phone may prompt, at a specific time interval (for example, one month), the user to collect a reference value of the preset physiological parameter again. FIG. 14A to FIG. 14D show an interface of a mobile phone according to some embodiments. The interface prompts the user to input the preset user information. The interface further includes collection prompt information of the reference value corresponding to the preset physiological parameter.

As shown in FIG. 14A, the mobile phone displays a first interface 30, and the first interface 30 is used to input the preset user information. In the first interface 30, the user may input information such as age, gender, whether the user has a history of chest pain (a typical type of chest pain of the coronary heart disease), whether the user has diabetes, whether the user has hypertension, whether the user has dyslipidemia, and whether the user smokes. The information except for age and gender may be optional. The first interface 30 further includes a first control 31 for collecting the reference value corresponding to the preset physiological parameter. In an example, after inputting the preset user information in the first interface 30, the user may trigger the first control 31 to enter a process of collecting the reference value corresponding to the preset physiological parameter.

The mobile phone may display a second interface 32 in response to the triggering operation performed by the user on the first control 31. The second interface 32 may be used to prompt the user to make preparations for collection of the reference value of the preset physiological parameter. For example, the second interface 32 may include two collection preparations: 1. Wear your smart watch as follows. 2. Stay still for at least 3 minutes. In addition, the second interface 32 further provides a schematic diagram for wearing the smart watch. The second interface 32 further includes a second control 33. Specific content of the second control 33 includes: "Ready for collection". After correctly wearing the smart watch and staying still for 3 minutes, the user may trigger the second control 33. In response to the triggering operation performed by the user on the second control 33, the mobile phone may display a third interface 34. The third interface 34 may display prompt information: "Collecting the reference value corresponding to the preset physiological parameter". In addition, the mobile phone sends a collection instruction to the smart watch, to instruct the smart watch to start to collect a parameter value corresponding to the preset physiological parameter.

After completing collection, the smart watch may return the collected parameter value to the mobile phone. After receiving the parameter value returned by the smart watch, the mobile phone may display a fourth interface 35. In addition, the fourth interface 35 may display prompt information of collection completion. In addition, the mobile phone may further save the received parameter value as the reference value of the preset physiological parameter.

In some embodiments, the mobile phone may implement the physiological parameter monitoring function by using a preset application. That the user uses a physiological parameter monitoring function of the mobile phone for the first time may specifically correspond to that the user starts the preset application for the first time. For example, when detecting an operation of starting the preset application for the first time by the user, the mobile phone may display the first interface 30, to prompt the user to input related information, and collect a related parameter value.

To ensure accuracy of the reference value corresponding to the preset physiological parameter, generally, a physiological parameter value of the user in a healthy normal state is collected. Therefore, the reference value may usually be collected when the user is in a non-exercise state. In some embodiments, before collection of the reference value corresponding to the preset physiological parameter, the mobile phone may further prompt, by using an interface (for example, the second interface 32), the user to stay still for a period of time (for example, 3 minutes) before collection. In a process of collecting the preset physiological parameter, the mobile phone may further prompt, by using an interface (for example, the third interface 34), the user to remain in a still state.

In some embodiments, S302 specifically means that the mobile phone obtains the reference value and the preset user information from a storage path storing the reference value and the preset user information.

S303: Analyze the reference value corresponding to the preset physiological parameter, the preset user information, and the first parameter value to obtain an analysis result 1.

Generally, a physiological parameter has a corresponding indicator range indicating a health state, which may be denoted as a normal indicator range. When the physiological parameter is out of the normal indicator range, it may indicate that a body status may be abnormal. The normal indicator range of the physiological parameter may be set with reference to the preset user information and a reference value of the physiological parameter of the user. In some other embodiments, the normal indicator range of the physiological parameter may alternatively be set with reference to empirical data corresponding to the physiological parameter.

For example, the preset physiological parameter includes the PWV, and the first parameter value includes a PWV value. The greater the PWV value is, the greater the pulse wave velocity is. When arterial elasticity is good, the pulse wave velocity is slow, and when the arterial elasticity is poor, the pulse wave velocity is fast. In a non-exercise state, the pulse wave velocity can reflect human arterial elasticity to some extent. The arterial elasticity is closely related to a probability of the coronary heart disease, and the poorer the arterial elasticity is, the greater the probability of the coronary heart disease is.

In this embodiment of this application, an accelerometer of the smart watch may be used to detect whether the user is in an exercise state. When it is determined that the user is in a non-exercise state, the PWV value is measured. In some embodiments, that the PWV value is within a normal indicator range may specifically include: The PWV value is within a normal indicator range of the PWV. The normal indicator range of the PWV may be set with reference to the age of the user or with reference to the preset user information.

For example, the preset physiological parameter includes the heart rate, and the first parameter value may include a heart rate value, a heart rate increase degree, and a heart rate decrease rate. In a non-exercise state, heart rates of people with the chronic coronary syndrome have no obvious characteristic in the absence of an acute attack. A heart rate has characteristic changes in an acute attack of the chronic coronary syndrome or an attack of the acute coronary syndrome. Common manifestations of an acute attack of the chronic coronary syndrome or an attack of the acute coronary syndrome (briefly referred to as an attack of the coronary heart disease below) include: The heart rate gradually increases in the attack, and then gradually decreases, and both a heart rate increase degree and a heart rate decrease rate have specific characteristic manifestations. For example, in the attack of the coronary heart disease, the heart rate increases, and a heart rate recovery process lasts for a long time, that is, the heart rate decrease rate is small.

In this embodiment of this application, when it is determined that the user is in a non-exercise state, after it is detected that the heart rate increases, a heart rate increase degree and a heart rate decrease rate in a heart rate decrease process after the heart rate increases may be analyzed.

In some embodiments, that a first parameter value corresponding to the heart rate is within a normal indicator range may specifically include the following several cases: ① The heart rate in a non-exercise state is within a normal indicator range of the heart rate. ② The heart rate is out of the normal indicator range of the heart rate, and the heart rate increase degree is less than or equal to a preset heart rate increase degree. ③ The heart rate is out of the normal indicator range of the heart rate, and the heart rate decrease rate is greater than or equal to a preset heart rate decrease rate. Correspondingly, that the first parameter value corresponding to the heart rate is out of the normal indicator range includes: The heart rate increase degree is greater than the preset heart rate increase degree, and/or the heart rate decrease rate is greater than or equal to the preset heart rate decrease rate. In some specific embodiments, the preset heart rate increase degree may be set to 30 beats per minute (beat per minute, bpm). The preset heart rate decrease rate may be set to 18 bpm.

For example, the preset physiological parameter includes the HRV, and the first parameter value may include related parameters of the HRV, for example, a standard deviation of normal-to-normal intervals and a proportion of successive normal-to-normal intervals that differ by more than 50 ms. HRV of people with the coronary heart disease has typical characteristics compared with HRV of healthy people. For example, a standard deviation of normal-to-normal intervals of continuously monitored HRV of people with the coronary heart disease is relatively small, and a proportion of successive normal-to-normal intervals that differ by more than 50 milliseconds (ms) also has a characteristic manifestation.

In some embodiments, that a first parameter value corresponding to the HRV is within a normal indicator range may specifically include: The standard deviation of normal-to-normal intervals in a non-exercise state is greater than or equal to a preset standard deviation of normal-to-normal intervals, and the proportion of successive normal-to-normal intervals that differ by more than 50 ms is greater than or equal to a preset proportion.

For example, the preset physiological parameter includes the blood pressure, and the first parameter value may include parameter values such as a blood pressure value, a blood pressure increase degree, and a blood pressure decrease ratio per minute. The blood pressure has characteristic changes in the attack of the coronary heart disease. Common manifestations include: The blood pressure gradually increases in the attack of the coronary heart disease, and then gradually decreases, and a disease rate is slow.

In some embodiments, that a first parameter value corresponding to the blood pressure is within a normal indicator range may specifically include: In a non-exercise state, the blood pressure is within a normal indicator range of the blood pressure; or the blood pressure is out of the normal indicator range of the blood pressure, and the blood pressure increase degree is less than or equal to a preset blood pressure increase degree; or the blood pressure decrease ratio per minute is greater than or equal to a preset blood pressure decrease ratio. Correspondingly, that the first parameter value corresponding to the blood pressure is out of the normal indicator range includes: The blood pressure increase degree is greater than the preset blood pressure increase degree, and/or the blood pressure decrease ratio per minute is greater than or equal to the preset blood pressure decrease ratio. In some specific embodiments, the preset blood pressure increase degree may be set to 20 mmHg (mmHg). The preset blood pressure decrease ratio may be set to 3% per minute.

For example, the preset physiological parameter includes the respiratory rate, and the first parameter value may include a respiratory rate value. An increased respiratory rate often occurs in the attack of the coronary heart disease. In some embodiments, that a first parameter value corresponding to the respiratory rate is within a normal indicator range may specifically include: The respiratory rate in a non-exercise state is within a normal range of the respiratory rate.

In some embodiments, when the preset physiological parameter includes more than one of the PWV, the heart rate, the HRV, the blood pressure, and the respiratory rate, the method in the foregoing embodiment may be used in S303, to separately compare first parameter values corresponding to preset physiological parameters with their respective corresponding normal indicator ranges, so as to obtain the analysis result 1. In some embodiments, the analysis result 1 may be denoted as a first analysis result.

It can be learned from the descriptions in the foregoing embodiment that collection cycles and value output frequencies of the smart watch for the preset physiological parameters may be different. When the smart watch detects that a first parameter value corresponding to a preset physiological parameter changes greatly, another preset physiological parameter may not have been updated. Therefore, in some embodiments, after obtaining a first parameter value corresponding to a preset physiological parameter (for example, a first preset physiological parameter), the mobile phone may first analyze the first parameter value. If the first parameter value is out of a corresponding normal indicator range, and a degree by which the first parameter value is out of the corresponding normal indicator range is greater than a preset degree (for example, 20%), the mobile phone may instruct the smart watch to immediately collect a first parameter value corresponding to another preset physiological parameter (for example, a second preset physiological parameter). Then, the mobile phone may perform analysis based on first parameter values corresponding to all the preset physiological parameters, to obtain the analysis result 1. The first preset physiological parameter includes one or more of the preset physiological parameters, and the second preset physiological parameter includes a physiological parameter other than the first preset physiological parameter.

In some embodiments, S303 may specifically include: inputting the reference value corresponding to the preset physiological parameter, the preset user information, and the first parameter value into a preset prompt model 1 (which may be denoted as a second preset prompt model), and obtaining an output result 1 (which may be denoted as a second output result) of the preset prompt model 1. The second output result is used to represent a probability that the preset physiological parameter of the user is abnormal. The output result 1 is the foregoing analysis result 1. The preset prompt model 1 is a model determined through training based on a large quantity of sample data. The preset prompt model 1 may be used to evaluate whether the first parameter value is abnormal. The sample data may include age and gender of a plurality of users, whether the users have diabetes, whether the users have hypertension, whether the users have dyslipidemia, whether the users smoke, PWVs, heart rates, parameters of HRV (for example, SDNN and pNN50), blood pressure, respiratory rates, and changes in these indicators.

In some embodiments, the output result 1 of the preset prompt model 1 is a probability value (which may be denoted as a first probability value), and the probability value is used to represent a probability that the preset physiological parameter is abnormal. The greater the probability value in the output result 1 is, the greater the probability that the preset physiological parameter is abnormal is.

S304: Determine whether the analysis result 1 satisfies a preset condition 1.

In some embodiments, if the analysis result 1 satisfies the preset condition 1, it indicates that the analysis result 1 represents that the preset physiological parameter of the user is abnormal.

In some embodiments, the preset condition 1 may specifically include: whether the first parameter value is out of the normal indicator range. When there is one preset physiological parameter, if a first parameter value corresponding to the preset physiological parameter is out of a normal indicator range, it indicates that the analysis result 1 satisfies the preset condition 1. On the contrary, if the first parameter value is within the normal indicator range, it indicates that the analysis result 1 does not satisfy the preset condition 1.

When there are a plurality of preset physiological parameters, the preset condition 1 may specifically include: whether first parameter values corresponding to more than one of the PWV, the heart rate, the HRV, the blood pressure, and the respiratory rate are within normal indicator ranges. For example, if first parameter values corresponding to at least two or more preset physiological parameters are out of normal indicator ranges, it indicates that the analysis result 1 satisfies the preset condition 1. On the contrary, if there are less than two preset physiological parameters whose first parameter value is out of a normal indicator range, it indicates that the analysis result 1 does not satisfy the preset condition 1.

In some embodiments, if first parameter values corresponding to any two or more preset physiological parameters are out of normal indicator ranges, it may be determined that the analysis result 1 satisfies the preset condition 1. In another embodiment, alternatively, when detecting that first parameter values corresponding to at least three, at least four, or all of the preset physiological parameters in the analysis result 1 are out of normal indicator ranges, the mobile phone may determine that the analysis result 1 satisfies the preset condition 1.

In an embodiment in which the preset prompt model 1 is used to analyze the reference value corresponding to the preset physiological parameter, the preset user information, and the first parameter value in S303, and the output result 1 is a probability value, S304 may specifically include: determining whether the output result 1 is greater than a preset threshold 1 (which may be denoted as a third preset threshold). The preset threshold 1 may be determined according to the trained preset prompt model 1.

If the analysis result 1 does not satisfy the preset condition 1, it indicates that no abnormality is found in the first parameter value that corresponds to the preset physiological parameter and that is collected this time. That is, each preset physiological parameter of the user is normal. In this case, the mobile phone may not perform processing. Further, the mobile phone may continue to obtain a new parameter value corresponding to the preset physiological parameter, and continue to analyze the parameter value. As shown in FIG. 12, when a determining result in S304 is "no", the mobile phone may return to S301 for further execution.

If the analysis result 1 satisfies the preset condition 1, it indicates that the analysis result 1 represents that the preset physiological parameter of the user is abnormal. In this case, whether the abnormality of the preset physiological parameter is related to a target body part may be further determined with reference to a chest pain condition of the user. In this embodiment of this application, when a determining result in S304 is "yes", the mobile phone may perform S305.

S305: Give prompt information 1.

In some embodiments, the prompt information 1 is used to prompt the user to cooperate in input of preset data, so as to further analyze the reference value corresponding to the preset physiological parameter, the preset user information, and the first parameter value. In some embodiments, the prompt information 1 may be denoted as first prompt information.

In some embodiments, the preset data includes sense information of a target body part of the user. The sense information may be specifically pain information.

The target body part may be specifically a chest region. In this embodiment, the prompt information 1 may also be denoted as chest pain evaluation prompt information. In an example, the chest pain evaluation prompt information may specifically include: sound prompt information, indicator light prompt information, and/or vibration prompt information. The sound prompt information, the indicator light prompt information, and the vibration prompt information may be used to prompt the user to view the smart watch or the mobile phone. In another embodiment, the target body part may alternatively be another body part.

In some other embodiments, the chest pain evaluation prompt information may further include image prompt information.

In some other embodiments, the chest pain evaluation prompt information may alternatively include more than one of the image prompt information, the sound prompt information, and/or the vibration prompt information.

It can be learned from the descriptions in the foregoing embodiment that the physiological parameter monitoring function may be specifically implemented by using a preset application. In this embodiment, the chest pain evaluation prompt information may be used to prompt the user to start the preset application, and evaluate and input, based on a current body status, whether a chest pain symptom occurs and a specific manifestation of the chest pain symptom.

In some other embodiments, when the chest pain evaluation prompt information includes image prompt information, the image prompt information may be a chest pain evaluation input window. The chest pain evaluation input window is used for the user to evaluate and input, based on a current body status, whether a chest pain symptom occurs and a specific manifestation of the chest pain symptom.

In some embodiments, S305 may specifically include: giving the prompt information 1 by using the mobile phone and/or the smart watch. In an embodiment in which the chest pain evaluation prompt information is used to prompt the user to start the preset application and input related information, the chest pain evaluation prompt information given by the mobile phone may specifically include: Open the preset application and input related information. The chest pain evaluation prompt information given by the smart watch may specifically include: Use your mobile phone to view and start the preset application, and input related information.

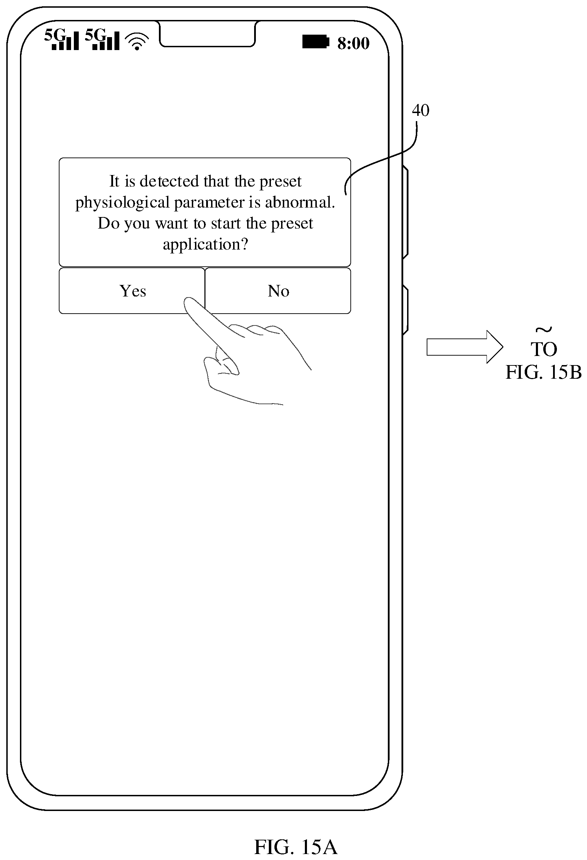

FIG. 15A to FIG. 15C show an interface in which the mobile phone gives the prompt information 1 according to some embodiments. When a determining result in S304 is "yes", the mobile phone may display image prompt information 40. The image prompt information 40 specifically includes: "It is detected that the preset physiological parameter is abnormal. Do you want to start the preset application?" In addition, the image prompt information 40 further includes two options: "Yes" and "No". When detecting a triggering operation performed by the user on the "Yes" option in the image prompt information 40, the mobile phone may start the preset application and display a chest pain evaluation input page 41. The chest pain evaluation input page displays "Chest pain evaluation: confirm whether you are experiencing chest pain based on your current physical condition". The chest pain evaluation input page 41 further includes a first option 42 and a second option 43. The first option 42 indicates that chest pain occurs, and the second option 43 indicates that chest pain does not occur.

If the mobile phone detects that the user triggers the first option 42 in the chest pain evaluation input page 41, it indicates that the user is currently experiencing chest pain. Further, the mobile phone may display a chest pain symptom evaluation interface 44. The chest pain symptom evaluation interface 44 may display "select an entry that fits your condition" to the user, and provide several different chest pain symptoms. A first symptom of chest pain includes: Discomfort occurs behind the sternum (including pressure, stuffiness, tightness, or heaviness in the chest). A second symptom of chest pain includes: The pain lasts no longer than 10 minutes. A third symptom of chest pain includes: The pain is induced by exercise or emotion, and can be relieved within minutes after rest or medication. The user may select, based on a current body status, one or more of the several chest pain symptoms provided by the chest pain symptom evaluation interface 44.

The chest pain symptom evaluation interface 44 may further include an "OK" option 45. After selecting an appropriate chest pain symptom entry based on the current body status, the user may trigger the "OK" option 45 for further evaluation.

In some other embodiments, the chest pain symptom evaluation interface may further provide the following information for the user to select: choices of a position of the pain (behind the sternum, upper abdomen, shoulder, arm, neck, back, side chest, or the like) based on a current body limb, a nature of the pain (pressure, stuffiness, tightness, heaviness, tingling, colic, or the like), duration of the pain (less than 1 minute, 1 minute to 10 minutes, or more than 10 minutes), a factor that induces the pain (exercise, intense emotions, or none), and whether the pain can be relieved within minutes after rest or medication (yes or no).

S306: Obtain the sense information of the target part input by the user.

For example, the target part is a chest region. After the user views the mobile phone and inputs whether a chest pain symptom occurs and a specific manifestation of the chest pain symptom according to the prompt information 1, the mobile phone may obtain chest pain information.

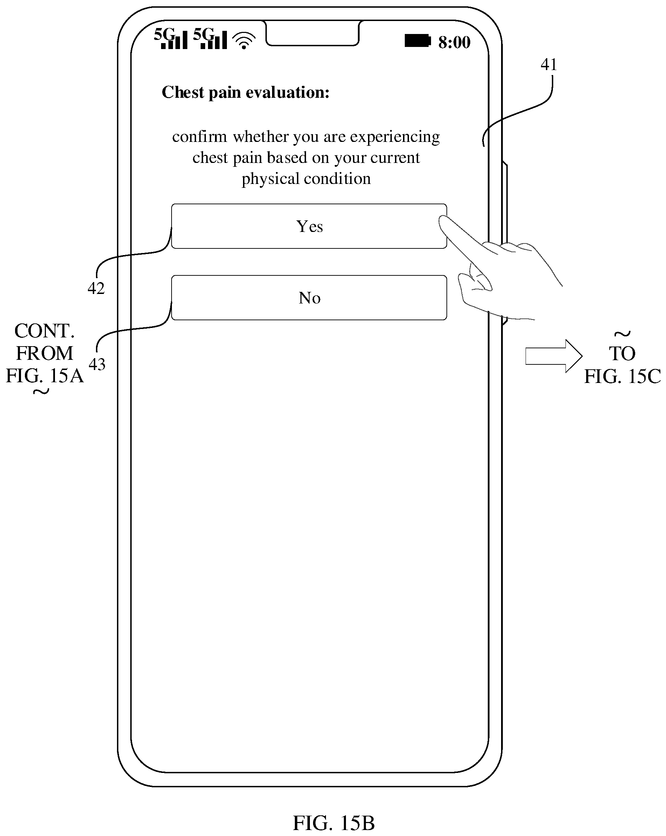

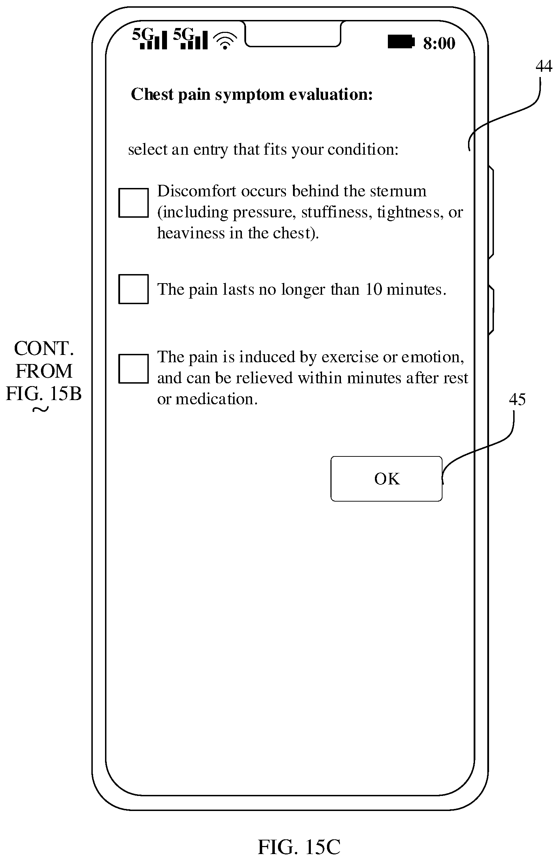

In some embodiments, after the mobile phone detects a triggering operation performed on the second option 43 in the chest pain evaluation input page 41 shown in FIG. 15B or a triggering operation performed on the "OK" option 45 in the chest pain symptom evaluation interface 44 shown in FIG. 15C, the mobile phone may obtain chest pain information of the user.

S307: Analyze the reference value corresponding to the preset physiological parameter, the preset user information, the sense information of the target part, and the first parameter value to obtain an analysis result 2.

In some embodiments, the analysis result 2 may be denoted as a second analysis result.

In some embodiments, S307 may specifically include: inputting the reference value corresponding to the preset physiological parameter, the preset user information, the sense information of the target part, and the first parameter value into a preset prompt model 2 (which may be denoted as a first preset prompt model), and obtaining an output result 2 (which may be denoted as a first output result) of the preset prompt model 2. The output result 2 is the foregoing analysis result 2. The preset prompt model 2 is a model determined through training based on a large quantity of sample data. The preset prompt model 2 may be used to evaluate whether the target part is abnormal. The first output result is used to represent a probability that the target body part of the user is abnormal.

In some embodiments, the output result 2 of the preset prompt model 2 is a probability value (which may be denoted as a second probability value), and the second probability value is used to represent a probability that the target part is abnormal. The greater the second probability value is, the greater the probability that the target part is abnormal is, or the greater the probability that the abnormality of the preset physiological parameter is associated with chest pain is.

S308: Determine whether the analysis result 2 satisfies a preset condition 2.

In some embodiments, the analysis result 2 may be denoted as a second analysis result. If the analysis result 2 satisfies the preset condition 2, it indicates that the target part of the user is abnormal.

In an embodiment in which the preset prompt model 2 is used to analyze the reference value corresponding to the preset physiological parameter, the preset user information, the sense information of the target part, and the first parameter value in S307, and the output result 2 is a second probability value, S308 may specifically include: determining whether the second probability value is greater than a preset threshold 2 (which may be denoted as a first preset threshold). The preset threshold 2 is used to represent a minimum value of the probability that the target part is abnormal. The preset threshold 2 may be determined according to the trained preset prompt model 2.

If the analysis result 2 satisfies the preset condition 2, the mobile phone may perform S309.

S309: Give prompt information 2, where the prompt information 2 is used to prompt the user to perform preset detection.

In this embodiment of this application, the prompt information 2 may be used to prompt the user to perform preset detection (for example, electrocardiogram detection). The smart watch shown in FIG. 3 to FIG. 5 can conveniently implement electrocardiogram detection. Specifically, the prompt information 2 may prompt the user to use the smart watch to perform electrocardiogram detection. In some embodiments, the prompt information 2 may be denoted as second prompt information.

In some embodiments, an urgency degree corresponding to the prompt information 2 is higher than an urgency degree corresponding to the prompt information 1.

In some embodiments, both the prompt information 1 and the prompt information 2 are in a sound prompt form. That an urgency degree corresponding to the prompt information 2 is higher than an urgency degree corresponding to the prompt information 1 may be specifically manifested in that prompt duration of the prompt information 2 is longer than prompt duration of the prompt information 1, or may be manifested in that prompt volume of the prompt information 2 is higher than prompt volume of the prompt information 1, or may be manifested in that prompt sound of the prompt information 2 is more urgent than prompt sound of the prompt information 1. In this way, an urgency degree can be indicated to the user more intuitively.

In some other embodiments, both the prompt information 1 and the prompt information 2 are in a vibration prompt form. That an urgency degree corresponding to the prompt information 2 is higher than an urgency degree corresponding to the prompt information 1 may be specifically manifested in that vibration duration of the prompt information 2 is longer than vibration duration of the prompt information 1, or may be manifested in that a vibration degree of the prompt information 2 is greater than a vibration degree of the prompt information 1. In this way, an urgency degree can be indicated to the user more intuitively.

In some other embodiments, both the prompt information 1 and the prompt information 2 are in an indicator light prompt form. That an urgency degree corresponding to the prompt information 2 is higher than an urgency degree corresponding to the prompt information 1 may be specifically manifested in that an indicator light blinking frequency of the prompt information 2 is greater than an indicator light blinking frequency of the prompt information 1, or may be manifested in that an indicator light color of the prompt information 2 is brighter than an indicator light color of the prompt information 1. In this way, an urgency degree can be indicated to the user more intuitively.

In this embodiment of this application, the mobile phone obtains, from the smart watch, a parameter value that corresponds to the preset physiological parameter of the user and that is collected by the smart watch in real time, and analyzes the parameter value to determine whether the preset condition 1 is satisfied. When the parameter value of the preset physiological parameter satisfies the preset condition 1, the user may be prompted to evaluate and input chest pain information. Then, whether the preset condition 2 is satisfied is analyzed with reference to the chest pain information. Finally, if it is determined that the preset condition 2 is satisfied, the mobile phone prompts the user to perform electrocardiogram detection. This can avoid a case in which a right time for electrocardiogram detection is missed because the user does not feel or ignores a chest pain symptom.

In addition, a preset threshold 3 (which may be denoted as a second preset threshold) may be further set for the preset prompt model 2. The preset threshold 3 is greater than the preset threshold 2. In S308, if the output result 2 (that is, the second probability value) of the preset prompt model 2 is greater than the preset threshold 2, the mobile phone may further compare the second probability value with the preset threshold 3. A determining result in S308 may be further divided into the following two cases: In a first case, the second probability value is greater than the preset threshold 2 and is less than or equal to the preset threshold 3. In a second case, the second probability value is greater than the preset threshold 2 and is greater than the preset threshold 3.

In some embodiments, in the first case, S309 may specifically include: if the second probability value is greater than the preset threshold 2 and is less than or equal to the preset threshold 3, giving the prompt information 2. The prompt information 2 may be used to prompt the user to perform preset detection.

In some other embodiments, in the second case, the second probability value is greater than the preset threshold 3, indicating that the target part may be abnormal. In this case, the mobile phone may give prompt information 3. The prompt information 3 is used to indicate that the target part is abnormal. In some embodiments, the prompt information 3 may be denoted as third prompt information. The prompt information 3 is used to indicate that the target body part of the user is abnormal. An urgency degree corresponding to the prompt information 3 is higher than the urgency degree corresponding to the prompt information 2. In some embodiments, the prompt information 3 may be specifically used to recommend the user to go to the hospital for treatment as soon as possible.

Alternatively, in some other embodiments, if it is determined that the second probability value is greater than the preset threshold 3, the mobile phone may display both the prompt information 2 and the prompt information 3. This makes it easy for the user to select a processing manner, and the user may choose to use the smart watch to perform electrocardiogram detection, or may choose to go to the hospital for treatment as soon as possible.

If the analysis result 2 does not satisfy the preset condition 2, it indicates that the target part of the user is normal. The abnormality of the preset physiological parameter may be caused by false detection. In this case, the mobile phone may give health prompt information.

S310: Give health prompt information.

In some embodiments, the health prompt information may be used to prompt the user to pay attention to a body status, a diet, moderate exercise, and the like. In another example, the health prompt information may be further used to inform the user that the mobile phone continuously monitors a change in a physiological parameter by using the smart watch. For example, the health prompt information may specifically include: 1. Pay attention to symptoms in the precordial region. If you experience significant discomfort, it is recommended to seek medical attention in time. 2. Maintain a healthy diet, engage in moderate exercise, and avoid strenuous activities and emotional excitement. 3. This device will continuously monitor changes in related physiological parameters.

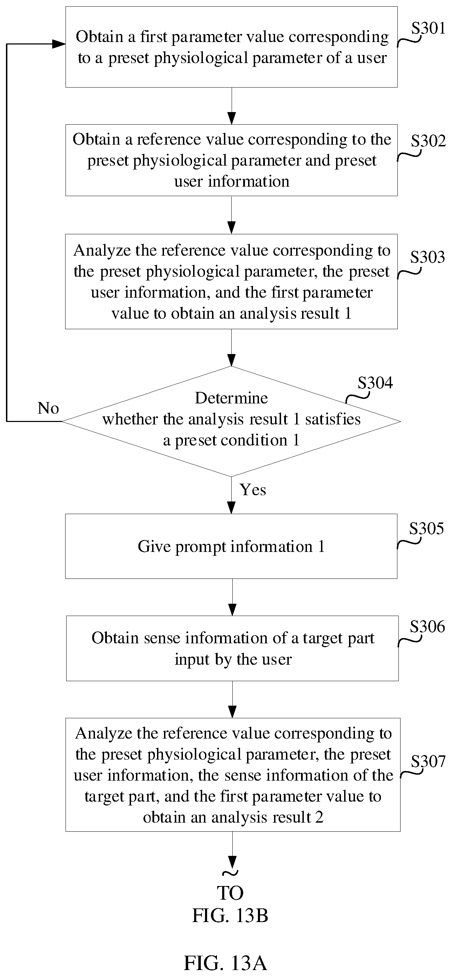

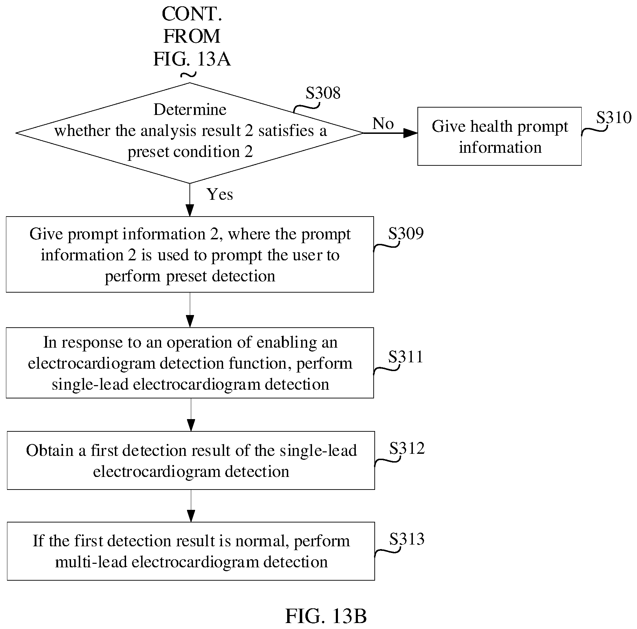

In some embodiments, after S309, the user may choose, according to the prompt information 1, to enable an electrocardiogram detection function. The electrocardiogram detection function can be divided into a single-lead electrocardiogram detection function and a multi-lead electrocardiogram detection function. Refer to FIG. 13A and FIG. 13B. In this embodiment, the foregoing method further includes the following steps:
S311: In response to an operation of enabling the electrocardiogram detection function, perform single-lead electrocardiogram detection.
S312: Obtain a first detection result of the single-lead electrocardiogram detection.
S313: If the first detection result is normal, perform multi-lead electrocardiogram detection.

Further, in some embodiments, that the mobile phone performs the multi-lead electrocardiogram detection may specifically include: The mobile phone first performs first multi-lead electrocardiogram detection, and obtains a second detection result of the first multi-lead electrocardiogram detection. Then, if the second detection result is normal, second multi-lead electrocardiogram detection is performed. Next, the mobile phone obtains a third detection result of the second multi-lead electrocardiogram detection. Finally, if the third detection result is normal, it indicates that the user is normal. In this case, the electronic device may give health prompt information. The multi-lead electrocardiogram detection includes the first multi-lead electrocardiogram detection and the second multi-lead electrocardiogram detection. In this way, the user may be sequentially prompted to perform electrocardiogram detection.

Further, in some embodiments, the method further includes: If the first detection result is abnormal, or the second detection result is abnormal, or the third detection result is abnormal, the mobile phone gives prompt information 4 (which may be denoted as fourth prompt information). The fourth prompt information is used to indicate that the detection result of the electrocardiogram detection is abnormal, and an urgency degree corresponding to the fourth prompt information is higher than the urgency degree corresponding to the first prompt information.

A posture used by the user to cooperate in the multi-lead electrocardiogram detection is more complicated than a posture used by the user to cooperate in the single-lead electrocardiogram detection. Therefore, the single-lead electrocardiogram detection is before the multi-lead electrocardiogram detection. If the result of the single-lead electrocardiogram detection is normal, the user may be prompted to pay attention to a body status. The user may choose to perform the single-lead electrocardiogram detection again, or choose whether to perform the multi-lead electrocardiogram detection. This can provide better comfort for the user.

In response to an operation performed by the user, the mobile phone may obtain an electrocardiogram after electrocardiogram detection is performed by using the smart watch. In some embodiments, the mobile phone may save the electrocardiogram based on an operation performed by the user.

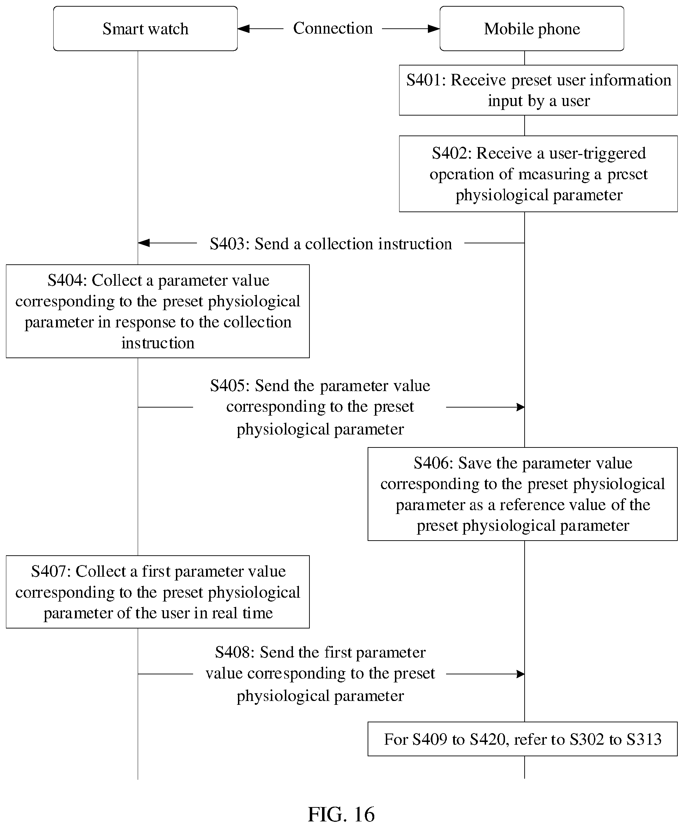

With reference to FIG. 16, the following describes a process in which the smart watch interacts with the mobile phone in the foregoing data processing method. A connection is established between the smart watch and the mobile phone.

S401: The mobile phone receives preset user information input by the user.

S402: The mobile phone receives a user-triggered operation of measuring a preset physiological parameter.

In an example, a specific implementation process of S401 and S402 may correspond to the user operation process shown in FIG. 14A to FIG. 14D. For example, after the user inputs the preset user information in the first interface 30, the mobile phone may receive the preset user information input by the user. After the user triggers the second control 33 in the second interface 32, that is, the mobile phone receives a user-triggered operation of measuring a preset physiological parameter, the mobile phone may perform S403 in response to the operation of measuring the preset physiological parameter.

S403: The mobile phone sends a collection instruction to the smart watch.

Correspondingly, the smart watch may receive the collection instruction sent by the mobile phone.

S404: The smart watch collects a parameter value corresponding to the preset physiological parameter in response to the collection instruction.

In S404, the parameter value collected by the smart watch based on the operation performed by the user on the mobile phone may be specifically the second parameter value in the foregoing embodiment. A specific implementation process in which the smart watch collects the parameter value corresponding to the preset physiological parameter is described in detail in a subsequent embodiment.

It should be noted that, the smart watch collects the parameter corresponding to the preset physiological parameter this time in response to the collection instruction, and the collection instruction is sent by the mobile phone in response to the user-triggered operation of measuring the preset physiological parameter. Generally, when the user triggers the operation of measuring the preset physiological parameter, the user makes related preparations, for example, wears the smart watch on a body part, and stays still for a period of time (for example, 3 minutes). Therefore, after receiving the collection instruction, the smart watch may immediately start to collect the parameter value corresponding to the preset physiological parameter, without a need for using the accelerometer to determine whether the user is in a non-exercise state and remains in the state for a period of time. In this way, waiting time after the user triggers the operation of measuring the preset physiological parameter can be reduced.

S405: The smart watch sends the parameter value corresponding to the preset physiological parameter to the mobile phone.

Correspondingly, the mobile phone receives the parameter value corresponding to the preset physiological parameter sent by the smart watch.

S406: The mobile phone saves the parameter value corresponding to the preset physiological parameter as a reference value of the preset physiological parameter.

The process in S401 to S406 may be a process in which the mobile phone interacts with the smart watch when the user uses the physiological parameter monitoring function for the first time. In some embodiments, after the mobile phone obtains and saves the preset user information of the user and the reference value of the preset physiological parameter, the mobile phone may enable the physiological parameter monitoring function. Then, the smart watch may monitor the physiological parameter of the user in real time, and transmit the physiological parameter to the mobile phone for analysis in time.

In the technical solution provided in this embodiment of this application, when the user uses the physiological parameter monitoring function for the first time, the mobile phone may obtain the preset user information. In addition, the mobile phone may instruct, in response to the operation performed by the user, the smart watch to collect the parameter value of the preset physiological parameter, and obtain the parameter value and save the parameter value as the reference value of the preset physiological parameter. This makes it easy for the mobile phone to evaluate a pretest probability of the coronary heart disease based on the reference value of the preset physiological parameter. In addition, this facilitates analysis of a real-time parameter value of the preset physiological parameter in a subsequent physiological parameter monitoring process.

For a process in which the smart watch interacts with the mobile phone after the mobile phone enables the physiological parameter monitoring function, refer to S407 to S416.

S407: The smart watch collects a first parameter value corresponding to the preset physiological parameter of the user in real time.

Collection cycles and value output frequencies for different preset physiological parameters may be different.

In some embodiments, the preset physiological parameter includes a PWV. Because the PWV is affected by a factor such as an exercise state, the user usually needs to be in a non-exercise state during PWV measurement. In some embodiments, the smart watch may determine, by using the accelerometer, whether the user is in an exercise state.

The smart watch starts timing after determining, by using the accelerometer, that the user enters a non-exercise state, and starts PWV measurement when timing time reaches first preset time. In this way, it can be ensured that a measured PWV value is not affected by an exercise state, and is more accurate. The first preset time may be set to 3 minutes, 4 minutes, 5 minutes, or the like. During PWV measurement, there is a need to collect PPG signals in a period of time, and then the PWV is calculated based on the PPG signals collected in the period of time.

In some embodiments, the PWV may be measured by using a single-lead ECG sensor and a PPG sensor. With reference to the structure shown in FIG. 3, it can be learned that the smart watch is provided with a single-lead ECG sensor and a PPG sensor.

In an example, after a related parameter is collected by using the single-lead ECG sensor and the PPG sensor, the PWV may be calculated according to the following formula: PWV=a/t1+b. Herein, t1 indicates a time difference between an R wave (a maximum positive peak in an ECG signal) in the ECG sensor and a subsequent adjacent first peak P1 in the PPG sensor. Herein, a and b may be empirical values. In some embodiments, both a and b may be values obtained through a large quantity of data training.

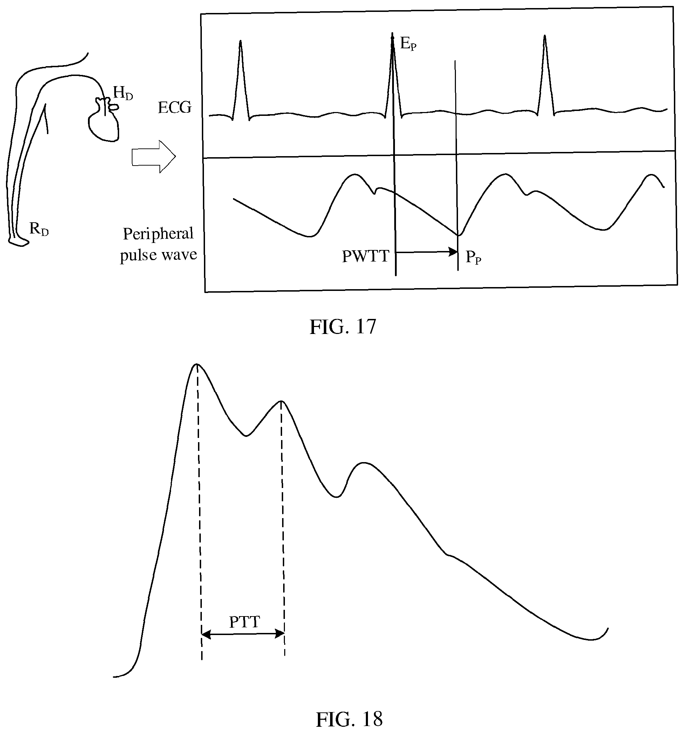

FIG. 17 and FIG. 18 are diagrams of a principle of measuring the PWV based on parameters collected by the single-lead ECG sensor and the PPG sensor. PWTT is the time it takes for an arterial pulse pressure wave to travel between a peripheral site and an aortic valve. Pulse transit time (PTT) is the time required for a pressure wave to travel between two arterial sites. In this method, time between two points of the arterial sites may be revealed, one point may be obtained from a waveform of the ECG sensor, and the other point may be obtained from a waveform of the PPG sensor.

In some other embodiments, the PWV may alternatively be measured by using the PPG sensor alone. In an example, after a related parameter is collected by using the PPG sensor, the PWV may be calculated according to the following formula: PWV=c/t2+d. Herein, t2 is a time difference between a first peak P1 and a second peak P2 in a waveform of the PPG sensor, and c and d are values obtained through a large quantity of data training. In another example, after a related parameter is collected by using the PPG sensor, the PWV may alternatively be obtained through machine learning with reference to analysis of a waveform of the PPG sensor.

In another embodiment, the PWV may be measured by using the PPG sensor in another manner.

In some embodiments, the smart watch may continuously collect PPG signals by using the PPG sensor, and output one PWV value at a specific time interval (for example, second preset time) based on PPG signals in the time interval. The second preset time may be set according to an actual situation, for example, the second preset time may be set to 10 minutes. That is, the smart watch outputs one PWV value every 10 minutes based on PPG signals in the 10 minutes. The foregoing manner may also be referred to as that the smart watch outputs one PWV value in one collection cycle, and one collection cycle is 10 minutes. Alternatively, the manner may be referred to as that a value output frequency of the smart watch for the PWV is outputting one PWV value every 10 minutes.

Only when the smart watch is worn on a body part of the user can a PPG signal be collected by using the PPG sensor. In some cases, if the smart watch is not worn on a body part of the user, the PPG sensor cannot be used to collect a PPG signal. Alternatively, if the smart watch is in poor contact with the body, quality of a PPG signal collected by using the PPG sensor is poor. In this case, a PWV value calculated based on the PPG signal may be inaccurate.

In addition, PPG signals in a continuous period of time are required for outputting a PWV value based on the PPG signals. For example, PPG signals in at least 30 consecutive seconds are required for outputting one PWV value.

In some specific embodiments, the smart watch collects a PPG signal in real time by using the PPG sensor, and outputs one PWV value every 10 minutes (that is, one collection cycle). Specifically, the smart watch selects, from PPG signals collected in 10 minutes, PPG signals in 30 consecutive seconds whose signal quality satisfies a quality requirement, to output one PWV value. That the signal quality satisfies the quality requirement may specifically include: The user is in a non-exercise state and remains in the state for at least 3 minutes. Preferably, if there are a plurality of groups of PPG signals in 30 consecutive seconds whose signal quality satisfies the quality requirement in 10 minutes, the smart watch may select one group of PPG signals in 30s whose signal quality is the best from the plurality of groups, and calculate and output a PWV value based on the group of PPG signals in 30s.

In an actual case, if no PPG signal satisfying the quality requirement is collected within 10 consecutive minutes in one collection cycle, a PWV value in the 10 minutes may be default.

In some embodiments, the preset physiological parameter includes a heart rate, and a corresponding parameter value may include a heart rate value. The heart rate value may be obtained through continuous monitoring by the PPG sensor. Because the heart rate is affected by a factor such as an exercise state, in some embodiments, the user usually needs to be in a non-exercise state during heart rate value measurement. It should be noted that for a specific implementation of measuring the heart rate value by using the PPG sensor, reference may be made to descriptions in a related technology, and details are not described in embodiments of this application.

The heart rate is a quantity of times a heart beats per minute, measured in beats per minute. In some embodiments, the smart watch may continuously collect PPG signals by using the PPG sensor, and determine and output one heart rate value every 1 minute. In an attack of the coronary heart disease, the heart rate value usually increases, and then gradually decreases, and a heart rate decrease rate is slow. Therefore, in some embodiments, the smart watch may record a plurality of heart rate values in a period of time, and analyze a heart rate change trend. Further, the smart watch may further store the heart rate change trend in the period of time. Further, the smart watch may calculate, at a specific time interval, information such as a heart rate increase degree, a heart rate increase rate, a heart rate decrease degree, and a heart rate decrease rate in the time interval, and send the data to the mobile phone. This makes it easy for the mobile phone to analyze whether the heart rate of the user is abnormal based on the foregoing information.

In some other embodiments, the smart watch may continuously send collected heart rate values to the mobile phone, and the mobile phone records a plurality of heart rate values in a period of time, and analyzes and stores a heart rate change trend. Then, the mobile phone may calculate, based on the plurality of heart rate values in the period of time, information such as a heart rate increase degree, a heart rate increase rate, a heart rate decrease degree, and a heart rate decrease rate in the period of time. This makes it easy for the mobile phone to analyze whether the heart rate of the user is abnormal based on the foregoing information.

When the preset physiological parameter includes HRV, the HRV may be obtained through continuous monitoring by the PPG sensor. It should be noted that for a specific implementation of measuring the HRV by using the PPG sensor, reference may be made to descriptions in a related technology, and details are not described in embodiments of this application.

In an attack of the coronary heart disease, related parameters of the HRV, for example, a standard deviation of normal-to-normal intervals and a proportion of successive normal-to-normal intervals that differ by more than 50 ms, have characteristic manifestations. The PPG sensor needs to perform monitoring for a continuous period of time to obtain each of the standard deviation of normal-to-normal intervals and the proportion of successive normal-to-normal intervals that differ by more than 50 ms. In some embodiments, the related parameters of the HRV (including the standard deviation of normal-to-normal intervals and the proportion of successive normal-to-normal intervals that differ by more than 50 ms) are calculated once every 1 minute, and a change trend is recorded, generated, and stored.

In some other embodiments, the preset physiological parameter includes blood pressure. The blood pressure may be calculated based on the PWV value calculated by using the PPG sensor. There is a positive correlation between the blood pressure and the PWV, and a conversion formula between the blood pressure and the PWV value may be obtained according to data training. After the PWV value is obtained, the blood pressure may be calculated according to the conversion formula between the blood pressure and the PWV value.

The blood pressure includes systolic blood pressure (systolic blood pressure, SBP) and diastolic blood pressure (diastolic blood pressure, DBP).

In some embodiments, a conversion formula between the systolic blood pressure and the PWV value may include: SBP=a1*PWV+b1. Herein, a1 and b1 may be values determined according to big data training. A conversion formula between the diastolic blood pressure and the PWV value may include: DBP=a2*PWV+b2. Herein, a2 and b2 may be values determined according to big data training.

In the foregoing method, a blood pressure value needs to be determined with reference to the PWV value. Therefore, a collection cycle and a value output frequency for the blood pressure need to be the same as a collection cycle and a value output frequency for the PWV. In some specific embodiments, a collection cycle of the smart watch for the blood pressure is 10 minutes, that is, a value output frequency of the smart watch for the blood pressure is outputting one blood pressure value every 10 minutes. If a PWV in a collection cycle is default, a blood pressure value in this collection cycle is also default.

In some other embodiments, the smart watch may also calculate a PWV value based on PPG signals in 30 consecutive seconds that satisfy a quality requirement in 10 minutes, and then calculate a blood pressure value based on the PWV value. In this way, the value output frequency of the smart watch for the blood pressure may be increased, and more blood pressure values are obtained, so as to better analyze whether the blood pressure of the user is abnormal. Specifically, after detecting that the user enters a non-exercise state and remains in the state for at least 3 minutes, the smart watch obtains a PPG signal to calculate a PWV value, so as to calculate a blood pressure value.

In an attack of the coronary heart disease, the blood pressure usually has the following manifestations: The blood pressure value increases, and then gradually decreases, and a blood pressure value decrease rate is slow. Therefore, in some embodiments, the smart watch may record a plurality of blood pressure values in a period of time, and analyze a blood pressure value change trend. Further, the smart watch may store the blood pressure value change trend in the period of time. Further, the smart watch may calculate, at a specific time interval, information such as a blood pressure increase degree, a blood pressure increase rate, a blood pressure decrease degree, and a blood pressure decrease rate in the time interval, and send the data to the mobile phone. This makes it easy for the mobile phone to analyze whether the blood pressure of the user is abnormal based on the foregoing information.

In some other embodiments, the smart watch may continuously send collected blood pressure values to the mobile phone, and the mobile phone records a plurality of blood pressure values in a period of time, and analyzes and stores a blood pressure change trend. Then, the mobile phone may calculate, based on the plurality of blood pressure values in the period of time, information such as a blood pressure increase degree, a blood pressure increase rate, a blood pressure decrease degree, and a blood pressure decrease rate in the period of time. This makes it easy for the mobile phone to analyze whether the blood pressure of the user is abnormal based on the foregoing information.

In addition, the preset physiological parameter may further include a respiratory rate, and a corresponding parameter value may include a respiratory rate value. The respiratory rate value may also be obtained through monitoring by the PPG sensor. In an example, the respiratory rate value may be calculated by using a machine learning model based on HRV and amplitude of a PPG signal that are measured by the PPG. The machine learning model is determined through training based on collected sample data. Similar to heart rate monitoring, respiratory rate values may be continuously monitored, and one respiratory rate value is output every 1 minute.

When the preset physiological parameter includes more than one of the PWV, the heart rate, the HRV, the blood pressure, and the respiratory rate, the smart watch may separately collect corresponding parameter values by using the methods in the foregoing embodiment.

S408: The smart watch sends the first parameter value corresponding to the preset physiological parameter to the mobile phone.

Correspondingly, the mobile phone may receive the first parameter value sent by the smart watch. This step may correspond to S301 in the procedure shown in FIG. 12, that is, the mobile phone obtains the first parameter value corresponding to the preset physiological parameter.

Collection cycles and value output frequencies for preset physiological parameters may be different. In some embodiments, after obtaining a first parameter value corresponding to one preset physiological parameter, the smart watch may send the first parameter value corresponding to the preset physiological parameter to the mobile phone. For example, the smart watch outputs one heart rate value and one respiratory rate value every 1 minute, and the smart watch sends a heart rate value and a respiratory rate value to the mobile phone once every 1 minute. The smart watch outputs one PWV value and one blood pressure value every 10 minutes, and the smart watch sends a PWV value and a blood pressure value to the mobile phone once every 10 minutes. Further, the mobile phone may determine, based on a heart rate value in 10 minutes, information such as a heart rate increase rate, a heart rate increase degree, a heart rate decrease degree, and a heart rate decrease rate in the 10 minutes. The respiratory rate is similar to the heart rate. In addition, the mobile phone may record, analyze, and store blood pressure values in a period of time (for example, 30 minutes or 1 hour) to obtain parameter values such as a blood pressure increase degree and a blood pressure decrease ratio per minute.

In some other embodiments, the smart watch may alternatively send parameter values of all the preset physiological parameters to the mobile phone after a new round of collection for all the preset physiological parameters. For example, the smart watch outputs one heart rate value and one respiratory rate value every 1 minute, and outputs one PWV value and one blood pressure value every 10 minutes. In other words, at least 10 minutes are required for the smart watch to obtain new parameter values of all the preset physiological parameters. In this embodiment, the smart watch may send parameter values corresponding to the preset physiological parameters to the mobile phone once every 10 minutes, and the parameter values sent each time include parameter values corresponding to all the preset physiological parameters. In the 10 minutes, the smart watch may record information about the heart rate value, such as a heart rate increase rate, a heart rate increase degree, a heart rate decrease degree, and a heart rate decrease rate. In addition, when sending the parameter values corresponding to the preset physiological parameters to the mobile phone, the smart watch sends the information such as the heart rate increase rate, the heart rate increase degree, the heart rate decrease degree, and the heart rate decrease rate in the 10 minutes to the mobile phone. The respiratory rate is similar to the heart rate.

S409: Obtain the reference value corresponding to the preset physiological parameter and the preset user information.

S410: Analyze the reference value corresponding to the preset physiological parameter, the preset user information, and the first parameter value to obtain an analysis result 1.

S411: Determine whether the analysis result 1 satisfies a preset condition 1.

S412: Give prompt information 1.

S413: Obtain sense information of a target part input by the user.

S414: Analyze the reference value corresponding to the preset physiological parameter, the preset user information, the sense information of the target part, and the first parameter value to obtain an analysis result 2.

S415: Determine whether the analysis result 2 satisfies a preset condition 2.

When the analysis result 2 satisfies the preset condition 2, S416 may be performed.

S416: Give prompt information 2, where the prompt information 2 is used to prompt the user to perform preset detection.

When the analysis result 2 does not satisfy the preset condition 2, S417 may be performed.

S417: Give health prompt information.

S418: In response to an operation of enabling an electrocardiogram detection function, perform single-lead electrocardiogram detection.

S419: Obtain a first detection result of the single-lead electrocardiogram detection.

S420: If the first detection result is normal, perform multi-lead electrocardiogram detection.

For a specific implementation process of S409 to S420, refer to descriptions of S302 to S313. Details are not described herein again.

In the technical solution provided in this embodiment of this application, the smart watch monitors and collects parameter values of some physiological parameters of the user in real time, and sends the parameter values to the mobile phone. The mobile phone may analyze, in real time with reference to these parameter values, whether the preset physiological parameters of the user are abnormal. In addition, when a specific condition is satisfied, the mobile phone gives prompt information to prompt the user to perform electrocardiogram detection. This avoids a case in which a right time for electrocardiogram detection is missed because the user does not feel or ignores a chest pain symptom.

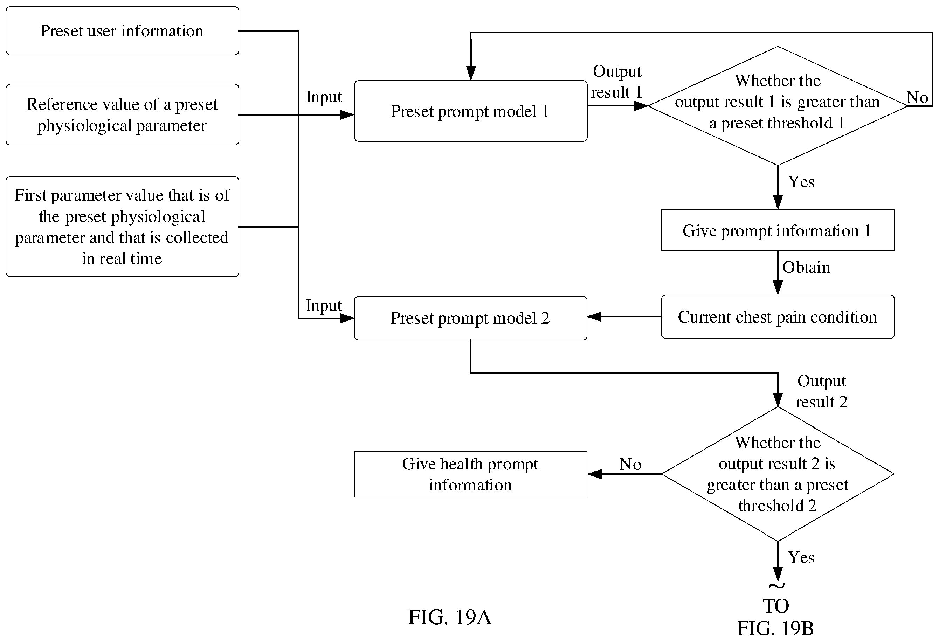

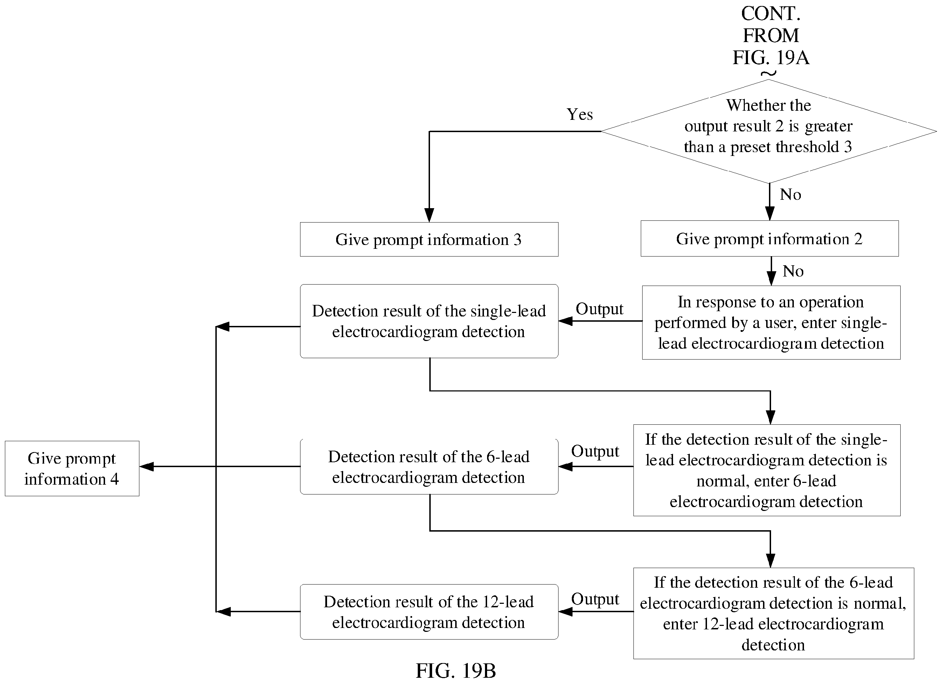

FIG. 19A and FIG. 19B show a complete procedure of a data processing method according to some embodiments. In a process in which a mobile phone monitors a physiological parameter of a user in real time, the mobile phone may obtain preset user information, a reference value of a preset physiological parameter, and a first parameter value that is of the preset physiological parameter and that is collected in real time, and input the three type of information into a preset prompt model 1. The preset prompt model 1 may output a result 1. Then, the mobile phone determines whether the output result 1 is greater than a preset threshold 1.

If the output result 1 is less than or equal to the preset threshold 1, it indicates that the first parameter value is normal. In this case, a new parameter value of the preset physiological parameter may be obtained, and is input with the preset user information and the reference value of the preset physiological parameter together into the preset prompt model 1, and then the newly obtained parameter value is analyzed.

If the output result 1 is greater than the preset threshold 1, it indicates that the preset physiological parameter is abnormal. In this case, the mobile phone may give prompt information 1. The prompt information 1 may be used to prompt the user to cooperate in input of preset data. For example, the preset data is chest pain information, and the user may input chest pain information of the user based on the prompt information 1. The mobile phone may obtain the chest pain information of the user.

Then, the mobile phone may input the preset user information, the reference value of the preset physiological parameter, the first parameter value that is of the preset physiological parameter and that is collected in real time, and the chest pain information into a preset prompt model 2. The preset prompt model 2 may output a result 2. Next, the mobile phone may determine whether the output result 2 is greater than a preset threshold 2.

If the output result 2 is less than the preset threshold 2, it indicates that there is no association between the abnormality of the preset physiological parameter and a chest region according to the preset user information, the reference value of the preset physiological parameter, the first parameter value, and the chest pain information, that is, there is no abnormality in the chest region. In this case, the mobile phone may give some health prompt information. In some embodiments, that the output result 2 is less than the preset threshold 2 may alternatively indicate that the user is less likely to suffer from an attack of the coronary heart disease.

If the output result 2 is greater than the preset threshold 2 and is less than or equal to a preset threshold 3, the mobile phone may give prompt information 2. The prompt information 2 may be used to prompt the user to perform electrocardiogram detection in time.

If the output result 2 is greater than the preset threshold 3, it indicates that the abnormality of the preset physiological parameter of the user may be associated with the chest region according to the preset user information, the reference value of the preset physiological parameter, the first parameter value, and the chest pain information. In some embodiments, that the output result 2 is greater than the preset threshold 3 indicates that the user is more likely to suffer from an attack of the coronary heart disease. In this case, the mobile phone may give prompt information 3. The prompt information 3 may be used to indicate that a target part (that is, the chest region) is abnormal. Further, the prompt information 3 may be used to recommend the user to go to the hospital for treatment as soon as possible. In some other embodiments, if the output result 2 is greater than the preset threshold 3, the mobile phone may give both the prompt information 2 and the prompt information 3 for the user to select a response manner.

When the user chooses, according to the prompt information 2, to perform electrocardiogram detection, the mobile phone may first perform single-lead electrocardiogram detection. Then, the mobile phone may obtain a detection result of the single-lead electrocardiogram detection. If the detection result of the single-lead electrocardiogram detection is normal, 6-lead electrocardiogram detection (for example, electrocardiogram detection of six limb leads) may be performed. The mobile phone may obtain a detection result of the 6-lead electrocardiogram detection. If the detection result of the 6-lead electrocardiogram detection is normal, 12-lead electrocardiogram detection may be performed. If a detection result of the 12-lead electrocardiogram detection is normal, health prompt information is given. It should be noted that the 12-lead electrocardiogram detection includes electrocardiogram detection of six limb leads and six precordial leads.

If the detection result of the single-lead electrocardiogram detection, the 6-lead electrocardiogram detection, or the 12-lead electrocardiogram detection is abnormal, prompt information 4 may be given. The prompt information 4 may be used to inform the user that the result of the electrocardiogram detection is abnormal. Further, the prompt information 4 may further prompt the user to go to the hospital for treatment as soon as possible.

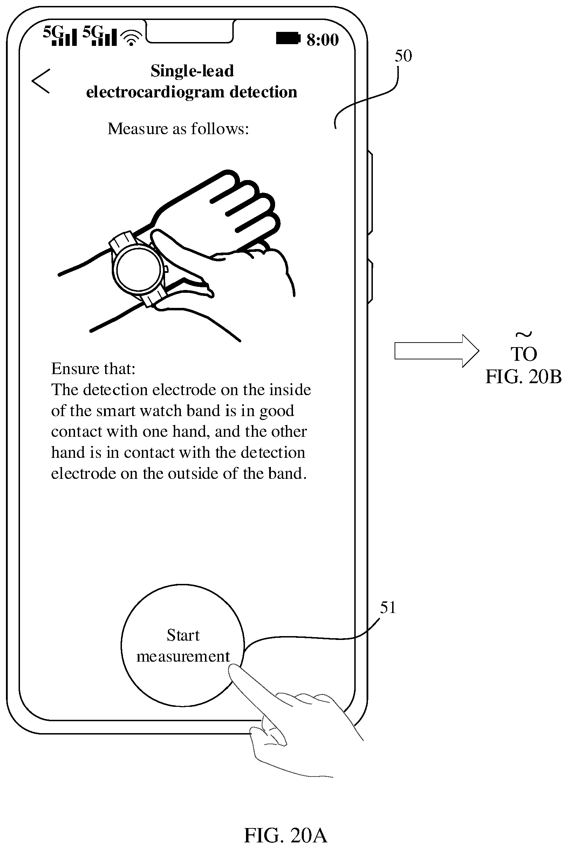

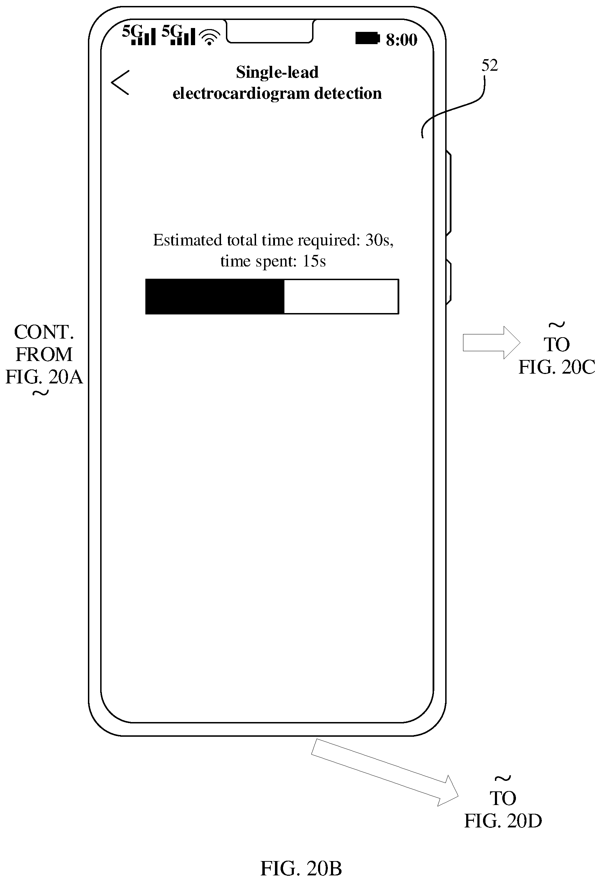

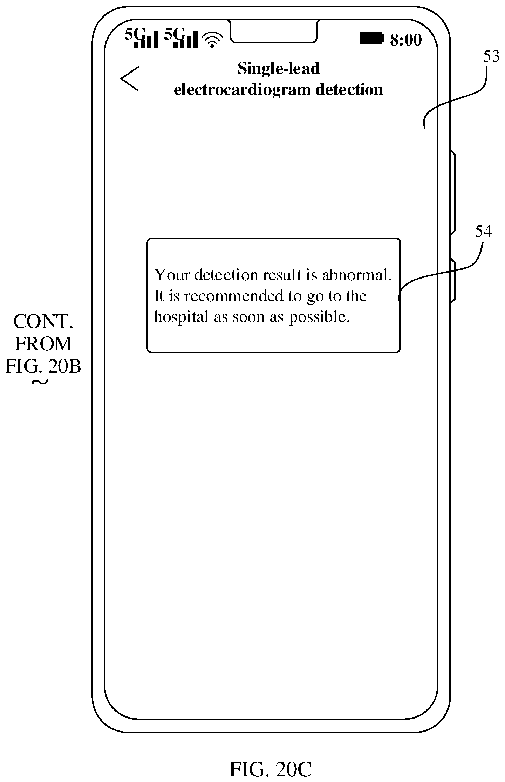

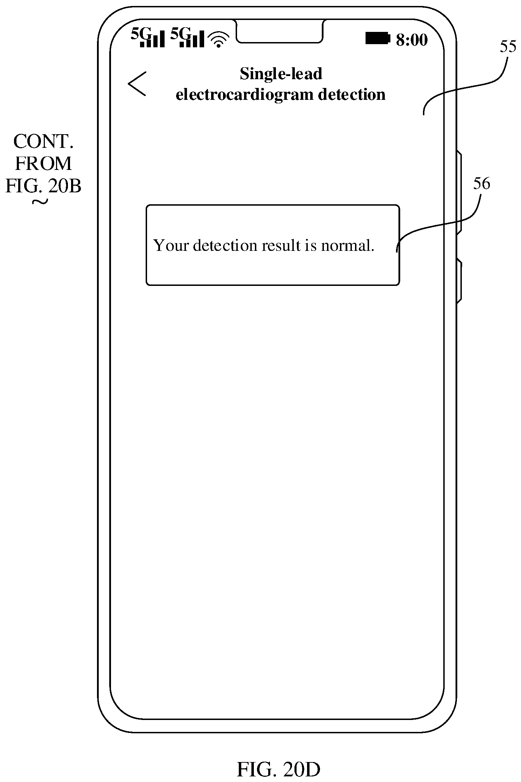

With reference to FIG. 20A to FIG. 22D, the following describes a process in which a user interacts with a mobile phone or a smart watch when the user chooses to perform electrocardiogram detection by using the smart watch.

FIG. 20A to FIG. 20D are schematic diagrams of an interface of a mobile phone in a process of performing single-lead electrocardiogram detection by using a smart watch. A text and an image are used in a guide interface 50 shown in FIG. 20A to display a detection manner for the single-lead electrocardiogram detection to the user. In addition, the guide interface 50 further displays prompt information: "Ensure that: The detection electrode on the inside of the smart watch band is in good contact with one hand, and the other hand is in contact with the detection electrode on the outside of the band." The guide interface 50 further includes a measurement control 51. After wearing the smart watch according to the information in the guide interface, the user may trigger the measurement control 51 in the guide interface 50.

In response to the triggering operation performed by the user on the measurement control 51, the mobile phone may instruct the smart watch to start to perform the single-lead electrocardiogram detection. In addition, the mobile phone may further display a prompt interface 52 of a measurement process, to display total time required for current measurement and time spent on the measurement to the user, for example, "Estimated total time required: 30s, time spent: 15s".

After the measurement is completed, the mobile phone may determine whether a measurement result is abnormal. If the measurement result is abnormal, the mobile phone may display a result page 53. The result page 53 may specifically include recommendation information 54: "Your detection result is abnormal. It is recommended to go to the hospital as soon as possible."

If the detection result of the single-lead electrocardiogram detection is normal, the mobile phone may display a result page 55. The result page 55 may specifically include a detection result 56: "Your detection result is normal."

Further, when the detection result of the single-lead electrocardiogram detection is normal, the mobile phone may further prompt the user to perform next detection, such as multi-lead electrocardiogram detection. In some embodiments, the result page 55 may further include a control for entering the next detection.

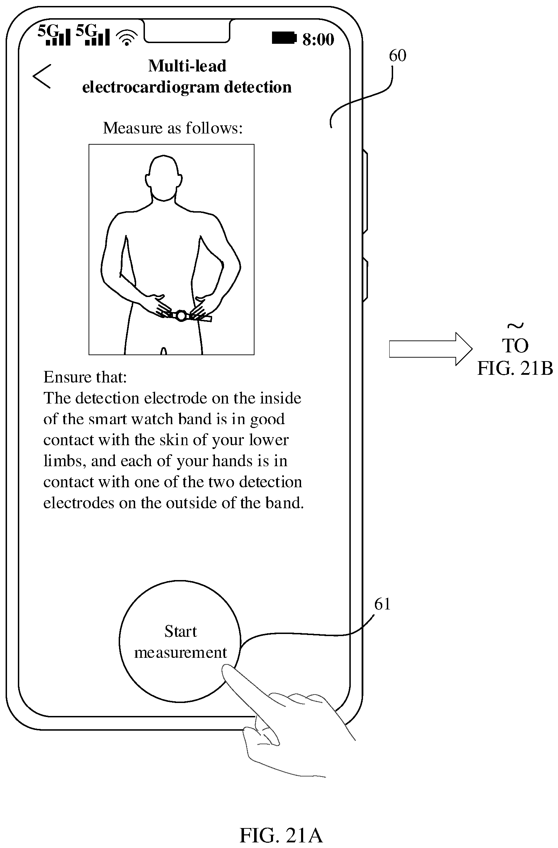

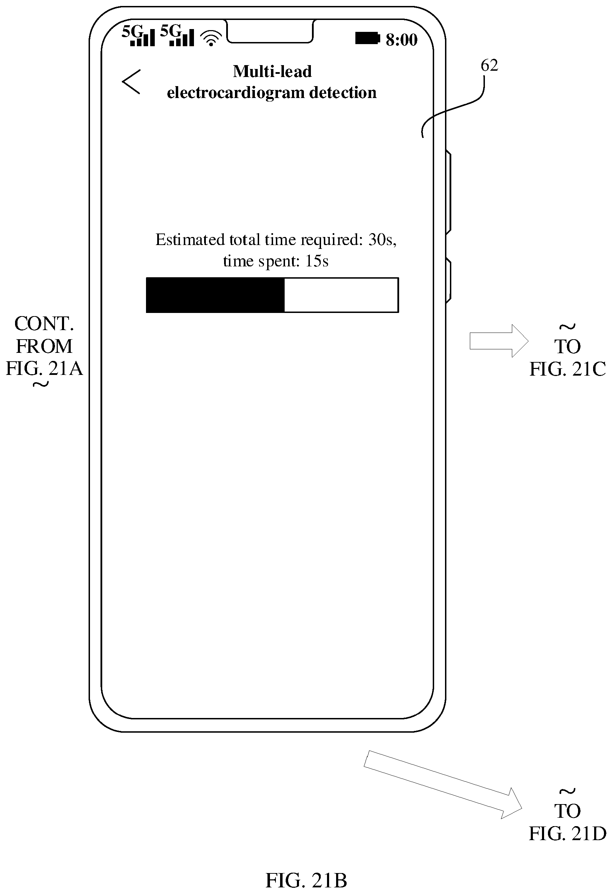

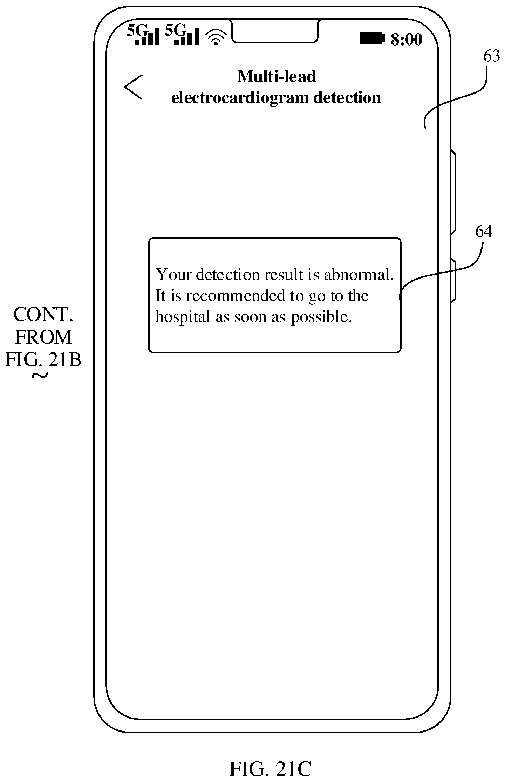

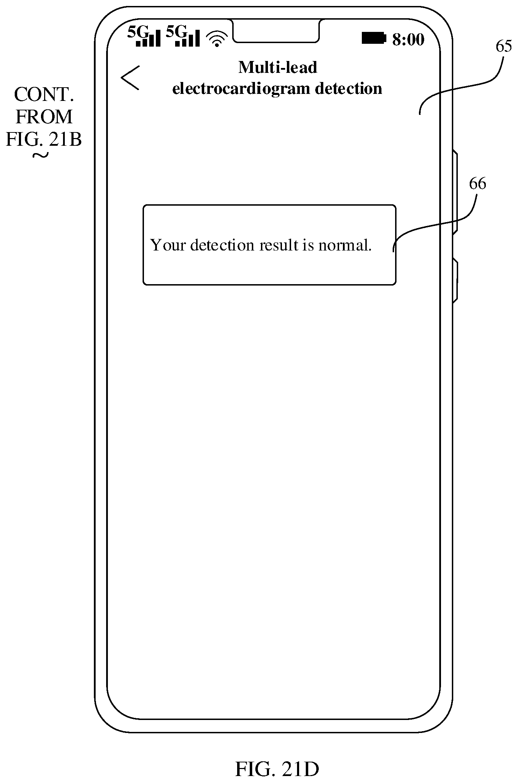

FIG. 21A to FIG. 21D are schematic diagrams of an interface of a mobile phone in a process of performing multi-lead electrocardiogram detection by using a smart watch. A text and an image are used in a guide interface 60 shown in FIG. 21A to display a detection manner for the multi-lead electrocardiogram detection to the user. In addition, the guide interface 60 further displays prompt information: "Ensure that: The detection electrode on the inside of the smart watch band is in good contact with the skin of your lower limbs, and each of your hands is in contact with one of the two detection electrodes on the outside of the band." The guide interface 60 further includes a measurement control 61. After wearing the smart watch according to the information in the guide interface, the user may trigger the measurement control 61 in the guide interface 60.

In response to the triggering operation performed by the user on the measurement control 61, the mobile phone may instruct the smart watch to start to perform the multi-lead electrocardiogram detection. In addition, the mobile phone may further display a prompt interface 62 of a measurement process, to display total time required for current measurement and time spent on the measurement to the user, for example, "Estimated total time required: 30s, time spent: 15s".

After the measurement is completed, the mobile phone may determine whether a measurement result is abnormal. If the measurement result is abnormal, the mobile phone may display a result page 63. The result page 63 may specifically include recommendation information 64: "Your detection result is abnormal. It is recommended to go to the hospital as soon as possible."

If the detection result of the multi-lead electrocardiogram detection is normal, the mobile phone may display a result page 65. The result page 65 may specifically include a detection result 66: "Your detection result is normal."

Further, when the detection result of the current multi-lead electrocardiogram detection is normal, the mobile phone may further prompt the user to perform next detection, such as next multi-lead electrocardiogram detection. In some embodiments, the result page 65 may further include a control for entering the next detection. With reference to the descriptions of FIG. 6 to FIG. 8, it can be learned that FIG. 21A to FIG. 21D specifically show a detection and interaction process of six limb leads, and the next multi-lead electrocardiogram detection may be detection of six precordial leads.

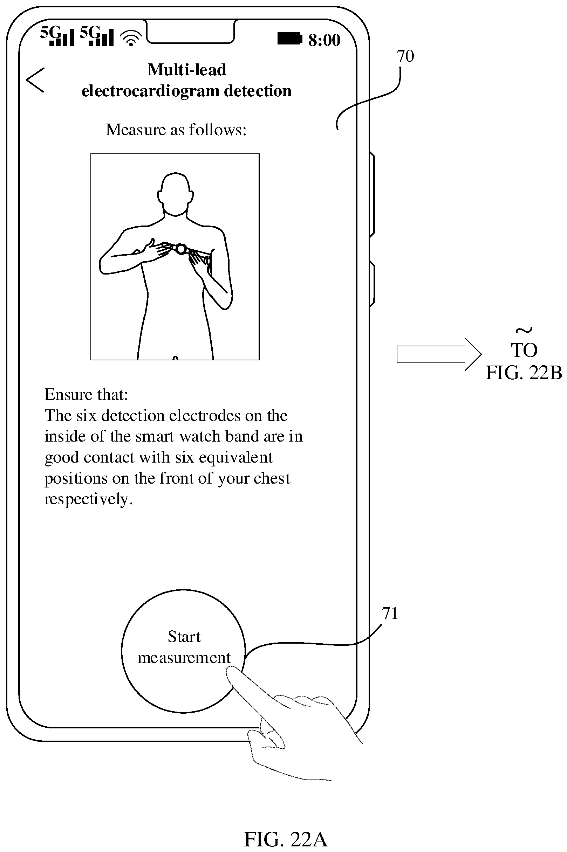

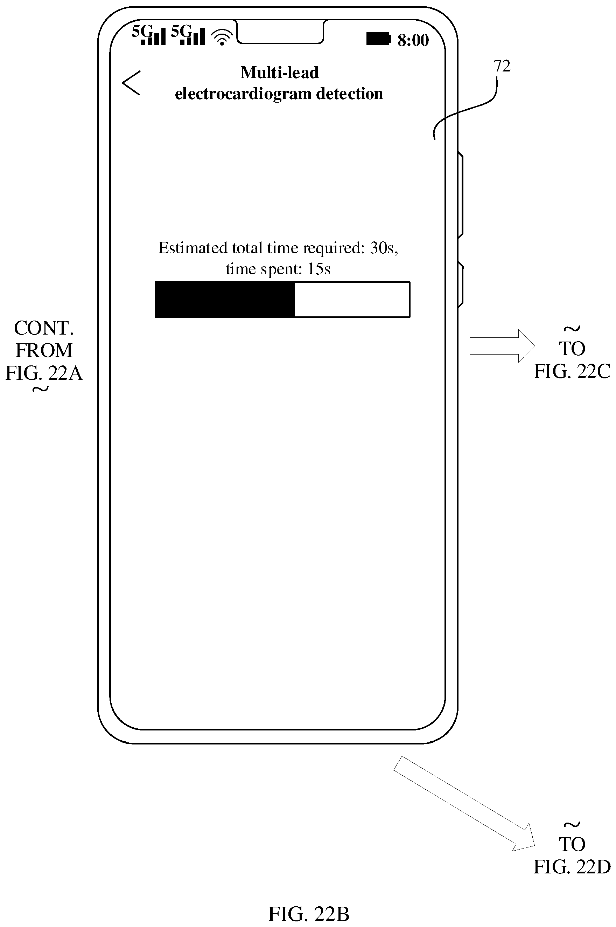

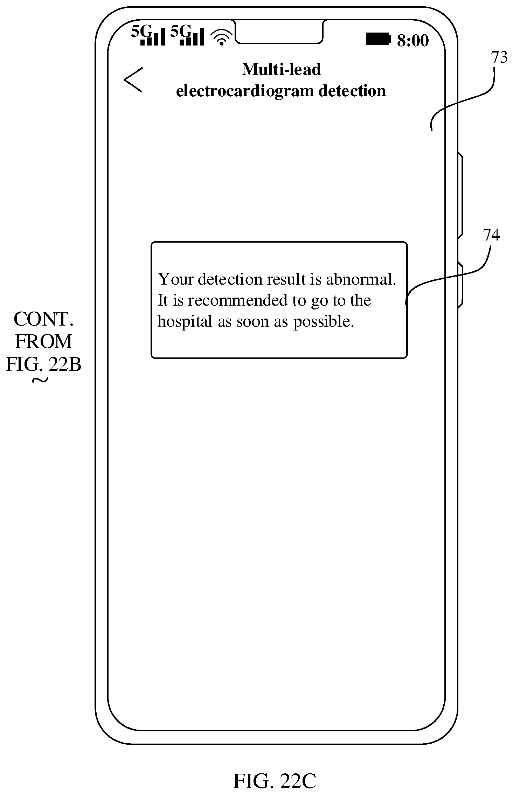

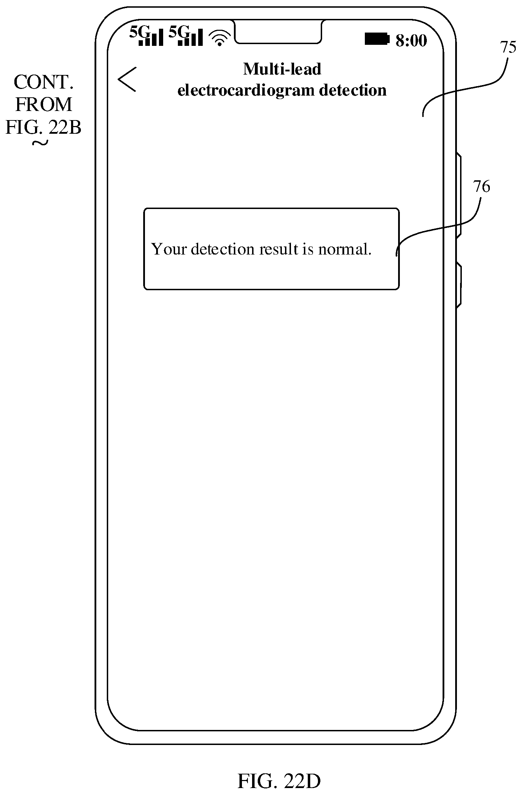

FIG. 22A to FIG. 22D are schematic diagrams of an interface of a mobile phone in a process of performing multi-lead electrocardiogram detection by using a smart watch. A text and an image are used in a guide interface 70 shown in FIG. 22A to display a detection manner for the multi-lead electrocardiogram detection to the user. In addition, the guide interface 70 further displays prompt information: "Ensure that: The six detection electrodes on the inside of the smart watch band are in good contact with six equivalent positions on the front of your chest respectively." The guide interface 70 further includes a measurement control 71. After wearing the smart watch according to the information in the guide interface, the user may trigger the measurement control 71 in the guide interface 70.

In response to the triggering operation performed by the user on the measurement control 71, the mobile phone may instruct the smart watch to start to perform the multi-lead electrocardiogram detection. In addition, the mobile phone may further display a prompt interface 72 of a measurement process, to display total time required for current measurement and time spent on the measurement to the user, for example, "Estimated total time required: 30s, time spent: 15s".

After the measurement is completed, the mobile phone may determine whether a measurement result is abnormal. If the measurement result is abnormal, the mobile phone may display a result page 73. The result page 73 may specifically include recommendation information 74: "Your detection result is abnormal. It is recommended to go to the hospital as soon as possible."

If the detection result of the current multi-lead electrocardiogram detection is still normal, the mobile phone may display a result page 75. The result page 75 may specifically include a detection result 76: "Your detection result is normal." In an embodiment in which the mobile phone prompts, by using a physiological parameter monitoring function, the user to perform electrocardiogram detection, if detection results of all electrocardiogram detection are normal, the mobile phone may further display health prompt information in the result page 75. For example, the health prompt information may specifically include: 1. Pay attention to symptoms in the precordial region. If you experience significant discomfort, it is recommended to seek medical attention in time. 2. Maintain a healthy diet, engage in moderate exercise, and avoid strenuous activities and emotional excitement. 3. This device will continuously monitor changes in related physiological parameters. If your symptoms worsen, you can take electrocardiogram measurement again or seek medical attention directly.

After electrocardiogram detection of 12 leads in total shown in FIG. 21A to FIG. 22D is completed, the mobile phone may obtain ECG waveforms separately obtained through the electrocardiogram detection of the 12 leads. The mobile phone may determine, based on related algorithms, whether the ECG waveforms are abnormal. Further, the mobile phone may interpret, by using an algorithm, the ECG waveforms obtained through the electrocardiogram detection, and may determine whether coronary heart disease or myocardial infarction attacks by identifying typical manifestations of the coronary heart disease or the myocardial infarction such as ST segment elevation/depression and pathological Q waves. Alternatively, the mobile phone may transmit the ECG waveforms of the electrocardiogram detection of the 12 leads to an institution with an ECG interpretation capability such as an Internet hospital for interpretation, and then obtain an interpretation result. Finally, the mobile phone gives related suggestions to the user according to a final interpretation result, such as seeking medical attention as soon as possible.

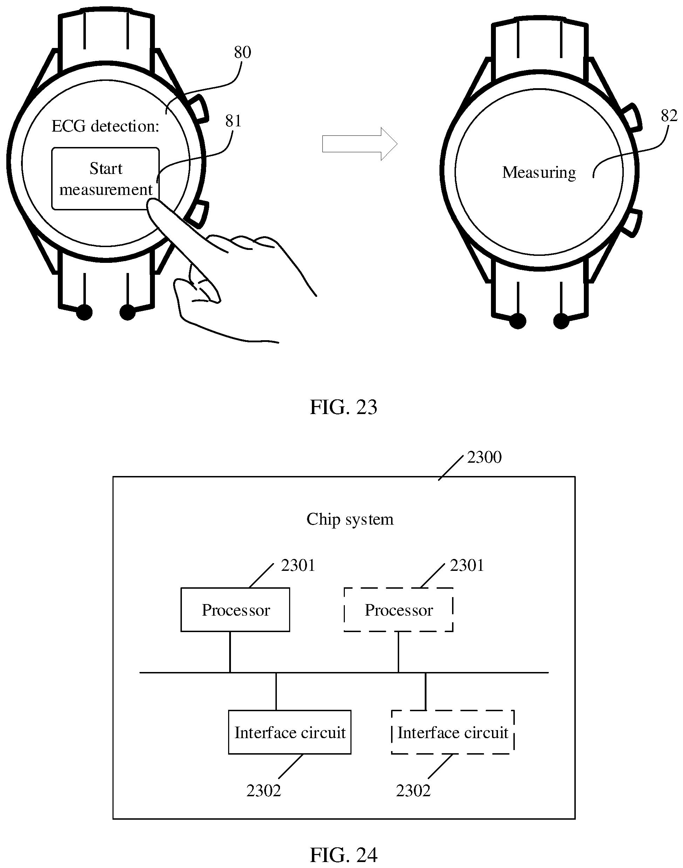

In the foregoing embodiment, the user triggers an electrocardiogram detection process on the mobile phone. In some other embodiments, guide information may alternatively be displayed on the smart watch, to instruct the user to enter an electrocardiogram detection process. As shown in FIG. 23, after the user chooses to enable an electrocardiogram detection function, the smart watch may display an interface 80. The interface includes a measurement control 81. In addition, when detecting a triggering operation (which may be a touch operation or a button press operation) performed by the user on the measurement control 81, the smart watch starts to perform electrocardiogram detection. In addition, the smart watch may display an interface 82 in an electrocardiogram detection process. It should be noted that, the user needs to bring corresponding detection electrodes of the smart watch into contact with body parts corresponding to the detection electrodes before the smart watch can execute the electrocardiogram detection function in response to a triggering operation performed by the user on the smart watch.

After completing an electrocardiogram detection process once, the smart watch may display information used to indicate measurement completion on a display screen of the smart watch. In an example, the smart watch may directly display a detection result of the current electrocardiogram detection on the display screen of the smart watch. In another example, the smart watch may alternatively display guide information for viewing a detection result. The guide information for viewing the detection result is used to prompt the user to view the detection result on the mobile phone.

It should be noted that, in an embodiment of this application, the mobile phone may monitor a preset physiological parameter of the user in real time by using the method shown in FIG. 12, and when a parameter value corresponding to the preset physiological parameter satisfies a specific condition, the user is prompted to evaluate a chest pain symptom. In addition, when the user selects chest pain information that matches a condition of the user in the chest pain symptom evaluation interface 44 shown in FIG. 15C, and triggers the "OK" option 45, the mobile phone may enter single-lead electrocardiogram detection, and display the guide interface 50 shown in FIG. 20A. In addition, when a detection result is normal, the mobile phone may successively instruct the user to perform the multi-lead electrocardiogram detection shown in FIG. 21A to FIG. 22D.

In addition, the user may actively choose to perform electrocardiogram detection by using the smart watch at any moment. Specifically, the user may choose to enable a single-lead electrocardiogram detection function on a preset application of the mobile phone. The mobile phone may also display the guide interface 50 shown in FIG. 20A, to instruct the user to perform the single-lead electrocardiogram detection. Similarly, the user may further choose to enable a multi-lead electrocardiogram detection function of six limb leads on the preset application of the mobile phone. The mobile phone may also display the guide interface 60 shown in FIG. 21A, to instruct the user to perform lead electrocardiogram detection of the six limb leads. The user may further choose to enable a multi-lead electrocardiogram detection function of six precordial leads on the preset application of the mobile phone. The mobile phone may also display the guide interface 70 shown in FIG. 22A, to instruct the user to perform multi-lead electrocardiogram detection of the six precordial leads.

Alternatively, when actively choosing to perform electrocardiogram detection by using the smart watch, the user may choose to enable a corresponding electrocardiogram detection function on the smart watch. The smart watch may display the interface 80 shown in FIG. 23, and the interface includes the measurement control 81. In addition, when detecting a triggering operation performed by the user on the measurement control 81, the smart watch displays the interface 82.

Some other embodiments of this application provide an electronic device. The electronic device may include a memory and one or more processors. The memory is coupled to the processor. The memory is configured to store computer program code. The computer program code includes computer instructions. When the processor executes the computer instructions, the electronic device may perform the functions or steps performed by the mobile phone in the foregoing method embodiments. For a structure of the electronic device, refer to the structure of the electronic device 100 shown in FIG. 2.

Some other embodiments of this application provide a wearable device. The wearable device may include a PPG sensor, a memory, and one or more processors. The PPG sensor and the memory are separately coupled to the processor. The PPG sensor is configured to collect a parameter value of a preset physiological parameter. The memory is configured to store computer program code. The computer program code includes computer instructions. When the processor executes the computer instructions, the wearable device may perform the functions or steps performed by the smart watch in the foregoing method embodiments. For a structure of the electronic device, refer to the structure of the wearable device 200 shown in FIG. 3.

In some other embodiments, the wearable device may further include an ECG sensor. The ECG sensor is configured to perform electrocardiogram detection. The ECG sensor is coupled to the processor.

Some other embodiments of this application further provide a communication system. The communication system includes an electronic device and a wearable device. The electronic device may establish a connection to the wearable device, and the electronic device may obtain, based on the connection, a parameter value that corresponds to a preset physiological parameter and that is collected by the wearable device.

An embodiment of this application further provides a chip system. As shown in FIG. 24, a chip system 2300 includes at least one processor 2301 and at least one interface circuit 2302. The processor 2301 may be interconnected to the interface circuit 2302 through a line. For example, the interface circuit 2302 may be configured to receive a signal from another apparatus (for example, a memory of a computer). For another example, the interface circuit 2302 may be configured to send a signal to another apparatus (for example, the processor 2301). For example, the interface circuit 2302 may read instructions stored in the memory and send the instructions to the processor 2301. When the instructions are executed by the processor 2301, the computer is enabled to perform the steps in the foregoing embodiments. Certainly, the chip system may further include another discrete device. This is not specifically limited in embodiments of this application.

An embodiment of this application further provides a computer-readable storage medium. The computer-readable storage medium includes computer instructions. When the computer instructions are run on the foregoing electronic device (for example, the mobile phone), the electronic device is enabled to perform the functions or steps performed by the mobile phone in the foregoing method embodiments.

An embodiment of this application further provides a computer program product. When the computer program product is run on a computer, the computer is enabled to perform the functions or steps performed by the mobile phone in the foregoing method embodiments. The computer may be an electronic device, such as a mobile phone.

Based on the descriptions of the foregoing implementations, a person skilled in the art may clearly understand that, for a purpose of convenient and brief description, division into the foregoing functional modules is used as an example for illustration. During actual application, the foregoing functions may be allocated to different functional modules and implemented according to a requirement. In other words, an inner structure of an apparatus is divided into different functional modules to implement all or some of the functions described above.

In the several embodiments provided in this application, it should be understood that the disclosed apparatus and method may be implemented in other manners. For example, the described apparatus embodiment is merely an example. For example, division into the modules or the units is merely logical function division and may be other division during actual implementation. For example, a plurality of units or components may be combined or integrated into another apparatus, or some features may be ignored or not performed. In addition, the displayed or discussed mutual couplings or direct couplings or communication connections may be implemented through some interfaces. The indirect couplings or communication connections between apparatuses or units may be implemented in electronic, mechanical, or other forms.

The units described as separate parts may or may not be physically separate, and parts displayed as units may be one or more physical units, may be located in one place, or may be distributed on different places. Some or all of the units may be selected according to actual requirements to achieve objectives of solutions of embodiments.

In addition, functional units in embodiments of this application may be integrated into one processing unit, each of the units may exist alone physically, or two or more units are integrated into one unit. The integrated unit may be implemented in a form of hardware, or may be implemented in a form of software functional unit.

When the integrated unit is implemented in the form of software functional unit and sold or used as an independent product, the integrated unit may be stored in a readable storage medium. Based on such an understanding, technical solutions in embodiments of this application essentially, or the part contributing to the conventional technology, or all or some of the technical solutions may be implemented in a form of software product. The software product is stored in a storage medium and includes several instructions for instructing a device (which may be a single-chip microcomputer, a chip, or the like) or a processor (processor) to perform all or some of the steps of the methods in embodiments of this application. The foregoing storage medium includes any medium that can store program code such as a USB flash drive, a removable hard disk, a read-only memory (read only memory, ROM), a random access memory (random access memory, RAM), a magnetic disk, or an optical disc.

The foregoing descriptions are merely specific implementations of this application, but are not intended to limit the protection scope of this application. Any variation or replacement within the technical scope disclosed in this application shall fall within the protection scope of this application. Therefore, the protection scope of this application shall be subject to the protection scope of the claims.

## Claims

1. A data processing method, wherein the method is applied to an electronic device, the electronic device establishes a connection to a wearable device, and the method comprises:
when the wearable device is worn on a body part of a user, obtaining, based on the connection, a first parameter value that corresponds to a preset physiological parameter of the user and that is collected by the wearable device, wherein the preset physiological parameter comprises at least one of the following: a pulse wave velocity PWV, a heart rate, heart rate variability HRV, blood pressure, and a respiratory rate;
analyzing the first parameter value to obtain a first analysis result; and
if the first analysis result represents that the preset physiological parameter of the user is abnormal, giving first prompt information, wherein the first prompt information is used to prompt the user to cooperate in input of preset data, so that new analysis is performed on the preset physiological parameter.

2. The method according to claim 1, wherein the preset data comprises sense information of a target body part of the user; and
after the giving first prompt information, the method further comprises:
obtaining the sense information of the target body part input by the user;
analyzing the sense information of the target body part and the first parameter value to obtain a second analysis result; and
giving second prompt information if the second analysis result represents that the target body part of the user is abnormal, wherein the second prompt information is used to prompt the user to perform preset detection by using the wearable device.

3. The method according to claim 2, wherein the analyzing the sense information of the target body part and the first parameter value to obtain a second analysis result comprises:
inputting the sense information of the target body part and the first parameter value into a first preset prompt model, wherein the first preset prompt model is determined through training; and
obtaining a first output result of the first preset prompt model, wherein the first output result is used to represent a probability that the target body part of the user is abnormal, and the second analysis result comprises the first output result; and
the giving second prompt information if the second analysis result represents that the target body part of the user is abnormal comprises:
if the first output result is greater than a first preset threshold and is less than or equal to a second preset threshold, giving the second prompt information, wherein the second preset threshold is greater than the first preset threshold.

4. The method according to claim 3, wherein the method further comprises:
if the first output result is greater than the second preset threshold, giving third prompt information, wherein the third prompt information is used to indicate that the target body part of the user is abnormal, and an urgency degree corresponding to the third prompt information is higher than an urgency degree corresponding to the second prompt information.

5. The method according to any one of claims 1 to 4, wherein the analyzing the first parameter value to obtain a first analysis result comprises:
inputting the first parameter value into a second preset prompt model, wherein the second preset prompt model is determined through training; and
obtaining a second output result of the second preset prompt model, wherein the second output result is used to represent a probability that the preset physiological parameter of the user is abnormal, and the first analysis result comprises the second output result; and
that the first analysis result represents that the preset physiological parameter of the user is abnormal comprises: the second output result is greater than a third preset threshold.

6. The method according to any one of claims 1 to 4, wherein the electronic device stores a reference value corresponding to the preset physiological parameter and preset user information; and
the analyzing the first parameter value to obtain a first analysis result comprises:
analyzing the reference value, the preset user information, and the first parameter value to obtain the first analysis result.

7. The method according to claim 6, wherein the analyzing the reference value, the preset user information, and the first parameter value to obtain the first analysis result comprises:
with reference to the reference value and the preset user information, determining a normal indicator range corresponding to the preset physiological parameter; and
determining whether the first parameter value corresponding to the preset physiological parameter is out of the corresponding normal indicator range; and
that the first analysis result represents that the preset physiological parameter of the user is abnormal comprises:
first parameter values corresponding to at least a preset quantity of preset physiological parameters are out of corresponding normal indicator ranges.

8. The method according to claim 7, wherein preset physiological parameters each correspond to a different value output frequency, and the analyzing the first parameter value to obtain a first analysis result comprises:
analyzing first parameter values obtained in a same cycle to obtain the first analysis result, wherein the electronic device obtains, in the same cycle, parameter values corresponding to the preset physiological parameters; and
the method further comprises: when it is detected that a first parameter value corresponding to a first preset physiological parameter is out of a corresponding normal indicator range, immediately obtaining a third parameter value corresponding to a second preset physiological parameter by using the wearable device, wherein the first preset physiological parameter comprises one or more of the preset physiological parameters, and the second preset physiological parameter comprises a physiological parameter other than the first preset physiological parameter;
analyzing the reference value, the preset user information, the first parameter value corresponding to the first preset physiological parameter, and the third parameter value to obtain a third analysis result; and
if the third analysis result represents that the preset physiological parameter of the user is abnormal, giving the first prompt information.

9. The method according to any one of claims 2 to 4, wherein after the giving second prompt information, the method further comprises:
in response to an operation of enabling an electrocardiogram detection function, performing single-lead electrocardiogram detection;
obtaining a first detection result of the single-lead electrocardiogram detection; and
if the first detection result is normal, performing multi-lead electrocardiogram detection.

10. The method according to claim 9, wherein the performing multi-lead electrocardiogram detection comprises:
performing first multi-lead electrocardiogram detection, wherein the multi-lead electrocardiogram detection comprises the first multi-lead electrocardiogram detection;
obtaining a second detection result of the first multi-lead electrocardiogram detection;
if the second detection result is normal, performing second multi-lead electrocardiogram detection, wherein the multi-lead electrocardiogram detection comprises the second multi-lead electrocardiogram detection;
obtaining a third detection result of the second multi-lead electrocardiogram detection; and
if the third detection result is normal, giving health prompt information.

11. The method according to claim 10, wherein the method further comprises:
if the first detection result is abnormal, or the second detection result is abnormal, or the third detection result is abnormal, giving fourth prompt information, wherein the fourth prompt information is used to indicate that the detection result of the electrocardiogram detection is abnormal, and an urgency degree corresponding to the fourth prompt information is higher than an urgency degree corresponding to the first prompt information.

12. The method according to any one of claims 2 to 4, wherein the electronic device stores a reference value corresponding to the preset physiological parameter and preset user information; and
the analyzing the sense information of the target body part and the first parameter value to obtain a second analysis result comprises:
analyzing the reference value, the preset user information, the sense information of the target body part, and the first parameter value to obtain the second analysis result.

13. A data processing method, wherein the method is applied to a wearable device, the wearable device establishes a connection to an electronic device, and the method comprises:
when the wearable device is worn on a body part of a user, collecting a first parameter value corresponding to a preset physiological parameter of the user; and
sending the first parameter value to the electronic device through the connection, wherein the first parameter value is to be analyzed by the electronic device to obtain a first analysis result, and if the first analysis result represents that the preset physiological parameter of the user is abnormal, first prompt information is given, wherein the first prompt information is used to prompt the user to cooperate in input of preset data, so that new analysis is performed on the preset physiological parameter.

14. A data processing method, wherein the method is applied to a wearable device, and the method comprises:
when the wearable device is worn on a body part of a user, collecting a first parameter value corresponding to a preset physiological parameter of the user, wherein the preset physiological parameter comprises at least one of the following: a pulse wave velocity PWV, a heart rate, heart rate variability HRV, blood pressure, and a respiratory rate;
obtaining a reference value corresponding to the preset physiological parameter and preset user information;
analyzing the first parameter value to obtain a first analysis result; and
if the first analysis result represents that the preset physiological parameter of the user is abnormal, giving first prompt information, wherein the first prompt information is used to prompt the user to cooperate in input of preset data, so that new analysis is performed on the preset physiological parameter.

15. An electronic device, wherein the electronic device comprises a processor and a memory, the memory is coupled to the processor, the memory stores computer program code, the computer program code comprises computer instructions, and when the computer instructions are executed by the processor, the electronic device is enabled to perform the method according to any one of claims 1 to 12 or the method according to claim 14.

16. A wearable device, wherein the wearable device comprises a photoplethysmography PPG sensor, a processor, and a memory, and the PPG sensor and the memory are separately coupled to the processor; and
the PPG sensor is configured to collect a parameter value corresponding to a preset physiological parameter, the memory stores computer program code, the computer program code comprises computer instructions, and when the computer instructions are executed by the processor, the wearable device is enabled to perform the method according to claim 13.

17. The wearable device according to claim 16, wherein the wearable device further comprises an electrocardiogram ECG sensor, the ECG sensor is coupled to the processor, and the ECG sensor is configured to perform electrocardiogram detection.

18. A communication system, wherein the communication system comprises the electronic device according to claim 15 and the wearable device according to claim 16 or 17, and the electronic device establishes a connection to the wearable device.

19. A computer-readable storage medium, comprising computer instructions, wherein when the computer instructions are run on an electronic device, the electronic device is enabled to perform the method according to any one of claims 1 to 12 or the method according to claim 14.
